(19) Europäisches Patentamt
European Patent Office
Office européen des brevets

(11) Publication number : **0 219 451 B1**

# (12) EUROPEAN PATENT SPECIFICATION

(45) Date of publication of patent specification : **20.03.91 Bulletin 91/12**

(21) Application number : **86810354.0**

(22) Date of filing : **12.08.86**

(51) Int. Cl.$^5$ : **C07D 213/80**, C07D 213/79, C07D 213/75, C07D 213/82, C07D 213/89, C07D 215/48, C07D 215/50, C07D 215/54, C07D 239/28, C07D 239/30, C07D 241/24

(54) **Semicarbazones and thiosemicarbazones.**

The file contains technical information submitted after the application was filed and not included in this specification

(30) Priority : **20.08.85 US 767465**

(43) Date of publication of application : **22.04.87 Bulletin 87/17**

(45) Publication of the grant of the patent : **20.03.91 Bulletin 91/12**

(84) Designated Contracting States : **AT BE CH DE FR GB IT LI LU NL SE**

(56) References cited :
**EP-A- 34 010**
**DE-A- 1 543 591**
**DE-A- 2 304 789**
**US-A- 3 753 680**
**US-A- 4 317 776**
**Chemical Abstracts, vol. 68, no. 7, February 12, 1968, Columbus, Ohio, USA F.Fujikawa et al. "Chemotherapeutics for Mycrobacterium tuberculosis" page 2862, column 2, abstract no. 29 557 b**

(73) Proprietor : **SANDOZ AG**
**Lichtstrasse 35**
**CH-4002 Basel (CH)**
**BE CH FR GB IT LI LU NL SE**
Proprietor : **SANDOZ-PATENT-GMBH**
**Humboldtstrasse 3**
**W-7850 Lörrach (DE)**
**DE**
Proprietor : **SANDOZ-ERFINDUNGEN Verwaltungsgesellschaft m.b.H.**
**Brunner Strasse 59**
**A-1235 Wien (AT)**
**AT**

(72) Inventor : **Anderson, Richard James**
**3367 Kenneth Drive**
**Palo Alto, CA 94303 (US)**
Inventor : **Leippe, Michael Mario**
**230 Madrone Avenue**
**Boulder Creek, CA 95006 (US)**

EP 0 219 451 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to novel substituted semicarbazones, thiosemicarbazones and isothiosemicarbazones, the synthesis thereof, the use of said compounds for the control of weeds, and compositions for weed control comprising such compounds.

EPA 0 034 010 discloses 2-formylbenzoic acid thiosemicarbazones and the use of such compounds as plant growth regulants.

US Patent 3,753,680 discloses phenyl-$CR=NR_1-C(Z)-NR_2R_3$ compounds, which may be substituted in the phenyl ring and wherein R is H or lower alkyl, that have herbicidal activity.

The compounds of the present invention are represented by the following formula (A) :

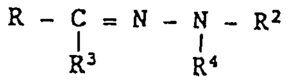

(A)

wherein R is phenyl or naphthyl, or is a heteroaromatic ring selected from

a) pyridyl which may be fused by its [b] or [c]-side with a benzene ring,

b) 2-pyridyl-N-oxide or 2-pyrazinyl-$N^1$-oxide,

c) pyrimidinyl,

d) pyrazinyl,

e) 3- or 4-cinnolinyl or 2-quinoxalinyl and

f) a five-membered heteroaromatic ring bound by one of its ring C-atoms to the $CR^3$-group selected from thienyl, furanyl, pyrrolyl, oxazolyl, thiazolyl, isoxazolyl, isothiazolyl, imidazolyl or pyrazolyl ; whereby said five-membered heteroaromatic ring may be fused by its [b]-side with a benzene ring ;

which group R is ortho-substituted by the group R′ and additionally bears one further substituent Y, both R′ and Y being attached to a ring carbon atom ;

R′ is the carboxy group in free form, salt form or ester form or is the group CO-$SR^6$ or CO-$NR^7R^8$,

Y is H, $C_{1-8}$alkyl, $C_{1-8}$haloalkyl, $C_{1-8}$alkoxy, $C_{1-8}$haloalkoxy, $C_{2-8}$alkenyloxy, $C_{2-8}$haloalkenyloxy, $C_{2-8}$alkynyloxy, phenyl, phenoxy, $C_{1-8}$alkylthio, OH, halogen, nitro or cyano ;

$R^2$ is the group $-C(=NR^{10})-SR^9$ or $-C(=X)-NHR^{10}$, each of $R^3$, $R^4$, $R^7$ and $R^8$ is independently hydrogen or $C_{1-8}$alkyl ; each of $R^6$ and $R^9$ is independently $C_{1-8}$alkyl,

$R^{10}$ is one of the groups

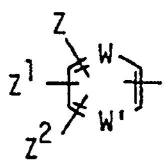

(G-1) ,

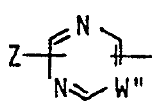

(G-2) ,

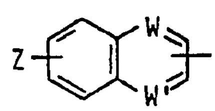

(G-3) or

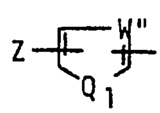

(G-4)

each of W, W′ and W″ is independently N or CH

$Q_1$ is oxygen, sulfur or NH

X is oxygen or sulfur

each of Z, $Z^1$ and $Z^2$ is independently one of the significances specified for – but independent of – Y, with the proviso that when R is phenyl mono-substituted in ortho by the carboxy group in free form or in salt form or by $C_{1-4}$alkoxy-carbonyl, then $R^3$ is $C_{1-8}$alkyl.

Where R′ is the carboxyl group in salt form, its cation is, for example, the cation of an alkali metal (e.g. the Li or Na cation) or of an earth alkali metal ; the ammonium cation ; a substituted ammonium cation (such as a $C_{1-5}$alkylammonium cation, a di-$C_{1-5}$alkylammonium cation, a tri-$C_{1-5}$alkylammonium cation, a tetra-$C_{1-5}$alkylammonium cation, a ($C_{1-5}$alkoxy-alkyl)ammonium cation, a (hydroxy-$C_{1-5}$alkoxy-$C_{1-5}$alkyl)ammonium cation, a (hydroxy$C_{1-5}$alkyl)ammonium cation) ; a phosphonium cation ; tri($C_{1-8}$alkyl)sulfonium cation or a tri($C_{1-8}$alkyl)sulfoxonium cation.

Where R′ is a carboxyl ester group (hereinafter $COOR^5$), then $R^5$ is for example $C_{1-8}$alkyl, $C_{1-8}$haloalkyl, $C_{2-10}$alkoxyalkyl, or $CH(R^{11})O-C(=X')-R^{12}$, wherein X′ is O or S, and $R^{11}$ and $R^{12}$ are, independently, H or $C_{1-8}$alkyl.

Where any of $R^3$, $R^4$, $R^5$, $R^6$, $R^7$, $R^8$, $R^9$, $R^{10}$, $R^{11}$, $R^{12}$, Y, Z, $Z^1$, $Z^2$ or the cation of the carboxyl group R′ is or comprises $C_{1-8}$alkyl, such alkyl group or moiety comprises preferably $C_{1-5}$, more preferably $C_{1-4}$ e.g. 1 or 2

carbon atoms.

$R^{12}$ is preferably $C_{1-8}$alkyl.

The terms $C_{2-8}$alkenyl-O and $C_{2-8}$alkynyl-O when used herein refer to $C_{2-8}$hydrocarbyloxy groups having 1 or 2, preferably 1, ethylenic bonds or 1 or 2, preferably 1, acetylenic bonds resp.

The terms $C_{1-8}$haloalkyl, $C_{1-8}$haloalkoxy and $C_{2-8}$haloalkenyloxy refer to $C_{1-8}$alkyl, $C_{1-8}$alkoxy and $C_{1-8}$alkenyloxy resp. substituted by one to six, particularly 1 to 3 halogen ; such halogen is preferably Cl or F.

It will be appreciated that heteroaromatic groups R which cannot be substituted at a ring carbon atom in ortho position relative to the bond by which R is tied to the $CR^3$ group of formula (A) are excluded by the above definition of R.

It will further be appreciated that, also by definition, whenever R is a bicyclic ring system comprising a benzene ring and a heteroring, said groug R is tied directly to the $CR^3$ group of formula (A) through a carbon ring atom of the heteroring moiety (i.e. not of the benzene moiety).

Where R has one of the above defined heteroaromatic significances, the substituted heteroring is particularly selected from pyridyl, quinolyl, 2-pyridyl-N-oxide, pyrimidinyl, pyrazinyl, thienyl or furyl, more particularly from pyridyl (4-, 3- or 2-pyridyl, preferably the latter) or thienyl (2- or 3-thienyl).

Where R is substituted by Y, the substituent is particularly halogen, $C_{1-5}$alkyl, $C_{1-5}$alkoxy or phenyl.

R' is preferably the carboxy group in free form, salt form or ester form.

A preferred significance of $R^5$ is $CH(R^{11})$-O-CO-$C_{1-8}$alkyl.

$R^7$ and $R^8$ are preferably $C_{1-8}$alkyl.

$R^2$ is preferably C(X)-NH$R^{10}$, more preferably CO-NH$R^{10}$.

$R^3$ is preferably $C_{1-8}$alkyl.

$R^{10}$ is preferably phenyl or pyridyl, which groups are conveniently unsubstituted or mono- or disubstituted.

Each of Y and Z conveniently signify independently H, halogen (F, Cl, Br, I), $C_{1-5}$alkyl, $C_{1-5}$alkoxy, $CF_3$, phenoxy, phenyl, nitro, cyano or hydroxy

$Z^1$ signifies conveniently H, halogen or $C_{1-5}$alkoxy.

$Z^2$ is preferably H.

The compounds of formula (A) are obtained by

a) reacting a compound of formula I

$$R\text{-}C(=O)\text{-}R^3 \quad I$$

wherein R and $R^3$ are as defined above with a compound of formula II

$$H_2N\text{-}NR^2R^4 \quad II$$

wherein $R^2$ and $R^4$ are as defined above, followed, where desired, by esterifying compounds of formula (A) wherein R' is the carboxyl group to compounds (A) wherein R' is a carboxyl-ester group, or

b) S-alkylating a compound of formula $A_1$

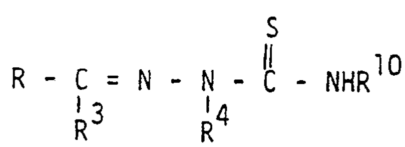

($A_1$)

wherein R, $R^3$, $R^4$ and $R^{10}$ are as defined above, with an alkylhalogenide of formula III

$$R^9Hal \quad III$$

wherein $R^9$ is as defined above and Hal is halogen, or a reactive functional derivative of said compound of formula III to give a compound of formula ($A_2$)

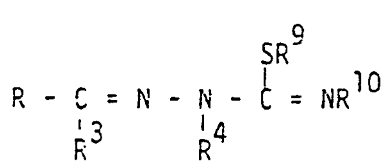

($A_2$)

wherein R, $R^3$, $R^4$, $R^9$, and $R^{10}$ are as defined above.

The reaction of compounds of formula (I) with compounds of formula (II) may be effected under the conditions known for the preparation of (thio)semicarbazones starting from the corresponding (thio)carbazides.

The reaction is conveniently carried out in a solvent which is inert under the reaction conditions, e.g. in an alcohol such as methanol or ethanol. An acid catalyst such as pyridyl tosylate may be added to promote the reaction. A suitable reaction temperature is room temperature or moderately enhanced temperature (e.g. between 10 and 40°C).

Compounds of formula (A) wherein R′ is $COOR^5$ may also be, and are in general preferably obtained by esterification of the corresponding compounds of formula (A) wherein R′ is the carboxyl group, in free form or in salt form, e.g. alkali metal salt form, using the desired esterification agent. Suitable esterification agents are alkylating agents such as $R^5$-halogenides, $R^5$-mesylates and $R^5$-tosylates or reactive functional derivatives thereof such as $CH_2N_2$.

The S-alkylation of thiosemicarbazones of formula ($A_1$) may be effected under the conditions known for the preparation of isothiosemicarbazones from thiosemicarbazones. In general, the reaction is carried out in a solvent which is inert under the reaction conditions, e.g. dimethylformamide. The compound of formula III is conveniently used as an iodide. It is in general advantageous to work in the presence of an acid binding agent, such as potassium carbonate. The R′ group may partially or completely be esterified or its eventual $R^5$ group interchanged, depending on the particular reaction conditions employed. Hydrolysis of such ester group followed, where desired, by treatment with an esterification (e.g. alkylation) agent for the introduction of the selected group $R^5$, will then result in the desired compound of formula ($A_2$).

Compounds of formula (A) wherein R′ is carboxyl may be converted to the corresponding salts in conventional manner and vice versa.

The compounds of formula (A) may be recovered from the reaction mixture in which they are formed by working up by established procedures.

The compounds of formula (A) may exist in either the syn or the anti form, although the anti form usually predominates.

The isothiosemicarbazones of the invention (see formula ($A_2$)) wherein $R^4$ is hydrogen may also exist in the corresponding isomeric form

$$RR^3\text{-}C{=}N\text{-}N{=}C(\text{-}SR^9)\text{-}NHR^{10}.$$

The starting materials and reagents employed in the process described herein are either known or, insofar as they are not known, may be produced in a manner analogous to the process described herein or to known processes.

The compounds of formula (A) have herbicidal activity as observed after their pre-emergent or post-emergent application to weeds or a weed locus.

The term "herbicide" (or "herbicidal") refers to an active ingredient (or an effect) which modifies the growth of plants because of plant growth regulating or phytotoxic properties so as to retard the growth of the plant or damage the plant sufficiently to kill it.

Application of a compound of formula (A) is made according to conventional procedure to the weeds or their locus using a herbicidally effective amount of the compound, usually from 100 g to 10 kg/ha.

The optimum usage of a compound of formula (A) is readily determined by one of oridinary skill in the art using routine testing such as green-house testing and small plot testing. It will depend on the compound employed, the desired effect (a phytotoxic effect requiring a higher rate than a plant growth regulating effect), the conditions of treatment and the like. In general satisfactory phytotoxic effects are obtained when the compound of formula (A) is applied at a rate in the range of from 0.2 to 5.0 kg, more preferably of from 0.25 to 2.5 kg per hectare.

While some of the compounds of formula (A), have activity on grass weeds, they demonstrate, in general, a higher level of herbicidal activity on broadleaf plants when applied post-emergence. Broadleaf plant (weed) species on which the compounds of the present invention show effective herbicidal activity include Brassica juncea, Amaranthus retroflexus, Abutilon theophrasti, Datura stramonium, Xanthium canadense, Cassia obtusifolia and Ipomoea purpurea.

When applied pre-emergence, the compounds of formula (A) demonstrate high levels of herbicidal activity on both broadleaf and grassy weeds.

The compounds of formula (A) may be advantageously combined with other herbicides for broadspectrum weed control. Examples of herbicides which can be combined with a compound of the present invention include those selected from the carbamates, thiocarbamates, chloroacetamides, dinitroanilines, benzoic acids, glycerol ethers, pyridazinones, uracils and ureas for controlling a broad spectrum of weeds.

The compounds of formula (A) are conveniently employed as herbicidal compositions in association with agriculturally acceptable diluents. Such compositions also form part of the present invention. They may contain, aside from a compound of formula (A) as active agent, other active agents, such as herbicides. They may be employed in either solid or liquid forms e.g. in the form of a wettable powder or an emulsifiable concentrate, incorporating conventional diluents. Such compositions may be produced in conventional manner, e.g. by mixing the active ingredient with a diluent and optionally other formulating ingredients such as surfactants.

The term diluents as used herein means any liquid or solid agriculturally acceptable material which may be added to the active constituent to bring it in an easier or improved applicable form, respectively to a usable or desirable strength of activity. It can for example be talc, kaolin, diatomaceous earth, xylene, or water.

Particularly formulations to be applied in spraying forms such as water dispersible concentrates or wettable powders may contain surfactants such as wetting and dispersing agents, e.g. the condensation product of formaldehyde with naphthalene sulphonate, an alkylarylsulphonate, a lignin sulphonate, a fatty alkyl sulphate, an ethoxylated alkylphenol and an ethoxylated fatty alcohol.

In general, the formulations include from 0.01 to 90% by weight of active agent and from 0 to 20% by weight of agriculturally acceptable surfactant, the active agent consisting either of at least one compound of formula (A) or mixtures thereof with other active agents. Concentrate forms of compositions generally contain between about 2 and 90%, preferably between about 5 and 70% by weight of active agent. Application forms of formulation may for example contain from 0.01 to 20% by weight of active agent.

The following examples are provided to illustrate the practice of the present invention. Temperature is given in degrees Centigrade. "RT" means room temperature. Parts and percentages are by weight. The symbols *, # and + when used in connection with melting points mean "gas", "softens" and "decomposes" resp.

## COMPOSITION EXAMPLES

### 1. Water dispersible powder

The sodium salt or isopropylammonium salt of compound 4-A (hereinafter, Table A) are dissolved to the desired percentage concentration in water containing 0.5% surfactant (e.g. a 1 : 1 : 1 mixture of sorbitan monolaurate : polyoxyethylene[20]sorbitane monolaurate : polyoxyethylene[20]sorbitan trioleate).

### 2. Suspension concentrate – 26%

26 Parts of th sodium salt of compound 30-A (Table A, hereinafter), 4 parts of propylene glycol and 1 part of octyl phenoxypoly[ethyleneoxy]ethanol are mixed and wet-milled to 5-10 micron particle size.

### 3. Wettable powder – 50%

50 Parts of the sodium salt of compound 30-A (Table A, hereinafter), 4 parts of sodium lignosulphonate, 1 part of sodium dialkylnaphthalene sulphonate and 45 parts of kaolin are mixed and air-milled. The mixture is added to water for spraying.

## EXAMPLE 1

To a solution of 2-acetylnicotinic acid (0.40 g, 2.4 mmol) in 7 ml of methanol is added a solution of 4-phenylsemicarbazide (0.37 g, 2.4 mmol) in 5 ml of methanol. The mixture is stirred at RT overnight, after which the solid precipitate is collected by filtration, washed with ethanol and dried to give 2-acetylnicotinic acid 4-phenylsemicarbazone, m.p. 174° (dec.) (compound 1, Table A).

## EXAMPLE 2

2-Acetylbenzoic acid (0.50 g, 3.0 mmol) and 2-methyl-4-(3-trifluoromethyl)phenylsemicarbazide (0.67 g, 3.0 mmol) are dissolved together in 15 ml of ethanol. After 3 hours at RT, 50 mg of pyridyl tosylate is added. After 2 more hours, the precipitated solid is collected by filtration and dried to give 2-acetylbenzoic acid 2-methyl-4-(3-trifluoromethyl)phenylsemicarbazone, m.p. 172° (gas) (compound 2, Table A).

## EXAMPLE 3

Following the procedure of either Example 1 or Example 2, each of the semicarbazone or thiosemicar-

bazone compounds 3-47 under Table A, 48-52 under Table B, 63-79 under Table C, 87-100 under Table D and 126-140 under Table E, is prepared from the corresponding semicarbazide or thiosemi- carbazide and acetyl compound.

**EXAMPLE 4**

To a solution of 2-acetylnicotinic acid 4-phenylsemicarbazone (0.32 g) in 5 ml of methanol is added 1 equivalent of sodium methoxide. The mixture is stirred at RT for 5 minutes, after which the solvent is removed by rotoevaporation to give the sodium salt of 2-acetylnicotinic acid 4-phenylsemicarbazone m.p. 203° (dec.).

Following the procedure of Example 4, each of the acids under Tables A, B, C, D and E is treated with 1 equivalent of sodium methoxide to give the corresponding sodium salt.

**EXAMPLE 5**

To 2-acetylnicotinic acid 4-phenylsemicarbazone (0.32 g) in 5 ml of methanol is added 1 equivalent of an aqueous solution of ammonium hydroxide. The mixture is stirred at RT for 5 minutes, after which the solvent is removed by rotoevaporation to give the ammonium salt of 2-acetylnicotinic acid 4-phenylsemicarbazone, m.p. 146-150° (softens) and 198-200° (gas).

Following the above procedure, each of the acids under Tables A, B, C, D and E is treated with 1 equivalent of aqueous ammonium hydroxide to give the corresponding ammonium salt.

**EXAMPLE 6**

Following the procedures of Example 5, using isopropylamine, 2-hydroxyethylamine or diisopropylamine, the following salts are obtained.

Of compound 1, the isopropylammonium salt, m.p. 94° (softens), 120°(gas)
Of compound 4, the isopropylammonium salt, m.p. 111-118°
Of compound 1, the 2-hydroxyethylammonium salt, m.p. 131° (gas)
Of compound 22, the 2-hydroxyethylammonium salt, m.p. 118° (gas)
Of compound 3, the diisopropylammonium salt, m.p. 208-210°
Of compound 4, the diisopropylammonium salt, m.p. 210-220°
(For the structure of compounds 1, 3, 4 and 22 see Table A, hereinafter).

TABLE A

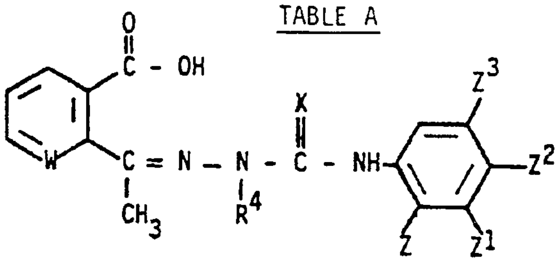

(Formula $A_3$)

| Cpd. No. | W | $R^4$ | X | Z | $Z^1$ | $Z^2$ | $Z^3$ | m.p.° acid | Na salt | $NH_4$ salt |
|---|---|---|---|---|---|---|---|---|---|---|
| 1 | N | H | O | H | H | H | H | 174* | 203 | 146#<br>200* |
| 2 | CH | $CH_3$ | O | H | $CF_3$ | H | H | 172* | 148* | |
| 3 | N | H | O | H | Cl | H | H | 153* | 205* | |
| 4 | N | H | O | H | F | H | H | 159* | 220#<br>232* | 140#<br>200* |
| 5 | N | H | O | H | $CF_3$ | H | H | 163-164 | 194* | |
| 6 | N | H | O | H | H | Cl | H | 227* | 213* | |
| 7 | N | H | O | Cl | H | H | H | 300 | 131-132* | |
| 8 | N | H | O | H | Cl | Cl | H | 163* | 189#<br>201* | |
| 9 | N | H | O | H | $CH_3$ | H | H | 166-167* | 211* | 192#<br>207* |
| 10 | N | H | O | H | H | F | H | 146-149*<br>208-220 | 170#<br>205* | |
| 11 | N | H | O | H | $OCH_3$ | H | H | 162-165* | 197* | |
| 12 | N | H | O | F | H | H | H | 162-165* | 197* | |
| 13 | N | H | S | H | H | H | H | 158-160 | | |
| 14 | N | H | S | H | Cl | H | H | 114-115* | 108+ | |
| 15 | N | H | S | F | H | H | H | 159-161* | 128-130* | |
| 16 | N | H | S | H | H | F | H | 173-174 | 159-163 | |
| 17 | N | H | S | H | Cl | Cl | H | 169-170 | 121-124* | |
| 18 | N | H | O | H | H | $O-C_6H_5$ | H | 148+ | 211* | |
| 19 | N | $CH_3$ | O | H | H | H | H | 98-100#<br>140* | 147-56*+ | 140-47 |
| 20 | N | $CH_3$ | O | H | Cl | H | H | 69#<br>140 | 118* | |

TABLE A (Continued)

| Cpd. No. | W | $R^4$ | X | Z | $Z^1$ | $Z^2$ | $Z^3$ | m.p.• acid | Na salt | $NH_4$ salt |
|---|---|---|---|---|---|---|---|---|---|---|
| 21 | N | $CH_3$ | O | H | F | H | H | 120-130 | 120# 140* | |
| 22 | CH | H | O | H | H | H | H | 174 | 184-86* | 150# |
| 23 | CH | H | O | H | $CF_3$ | H | H | 164* | 255 | |
| 24 | CH | H | O | H | Cl | H | H | 158* | 126* | 87# 140-147 |
| 25 | CH | H | O | H | F | H | H | 169* | 203* | |
| 26 | CH | H | O | H | $CF_3$ | Cl | H | 170* | 173* | |
| 27 | CH | H | S | H | H | H | H | 129-131 | 115# 134* | |
| 28 | CH | $CH_3$ | O | H | H | H | H | 177 | | |
| 29 | CH | $CH_3$ | O | H | H | $CH_3$ | H | 176-178* | | |
| 30 | N | H | O | H | Cl | H | Cl | 193+ | 222* | |
| 31 | N | H | O | H | F | H | F | 186* | 240* | |
| 32 | N | H | O | F | F | H | H | 168# 202 | 220* | |
| 33 | N | H | O | Cl | Cl | H | H | 168# 198 | 194* | |
| 34 | N | H | O | F | H | H | F | 154-157 | 234-235* | |
| 35 | N | H | O | F | H | H | $CH_3$ | 173# 203 | 226-227* | |
| 36 | N | H | O | H | $OCH_3$ | H | $OCH_3$ | 185* | 203 | |
| 37 | N | H | O | F | H | H | Cl | 167-171* | 204-206+ | |
| 38 | N | H | O | F | Cl | H | H | 152-154* | 200-202+ | |
| 39 | N | H | O | H | Cl | Cl | H | 163* | 189# | |
| 40 | N | H | O | H | $NO_2$ | H | H | 190+ | 194-195+ | |
| 41 | N | H | S | H | CN | H | H | 168-169 | 166# | |
| 42 | N | H | O | H | Br | H | H | 175* | 208-210* | |
| 43 | N | H | O | H | I | H | H | 174* | 212* | |
| 44 | N | H | O | H | OH | H | H | 204-210 | | |

TABLE A (Continued)

| Cpd. No. | W | $R^4$ | X | Z | $Z^1$ | $Z^2$ | $Z^3$ | m.p. acid | m.p. Na salt |
|---|---|---|---|---|---|---|---|---|---|
| 45 | N | H | O | H | $OC_6H_5$ | H | H | 164* | 206* |
| 46 | N | H | O | H | $C_2H_5$ | H | H | 178-80* | 200 #<br>218* |
| 47 | N | H | O | H | $OnC_4H_9$ | H | H | 172-75* | 207* |

TABLE B

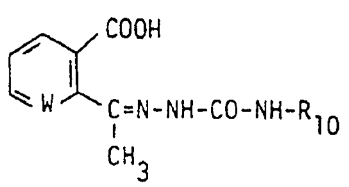

(Formula $A_4$)

| Cpd. | W | $R_{10}$ | m.p. acid | m.p. Na-salt |
|---|---|---|---|---|
| 48 | N | 2-pyridyl | 195-197° | 183° |
| 49 | N | 6-chloro-2-pyridyl | 171-172° | 190-194° |
| 50 | N | 3-pyridyl | 167° #<br>206-208° | 208°* |
| 51 | CH | 2-pyridyl | 128-129°* | 200°* |
| 52 | CH | 3-pyridyl | 152-153° | 175°+ |

## EXAMPLE 7

A suspension of 2-acetylnicotinic acid 4-phenylsemicarbazone (compound 1) (0.50 g) in 20 ml of methanol is treated with diazomethane until the yellow color of the solution persists. The solvent is removed in vacuo to give methyl 2-acetylnicotinate 4-phenylsemicarbazone, m.p. 177-178° (compound 53, Table C).

## EXAMPLE 8

A mixture of the sodium salt of 2-acetylnicotinic acid 4-(3-fluorophenyl)semicarbazone (compound 4) (1.0 g, 3.0 mmol) and chloromethyl acetate (0.4 g, 3.5 mmol) in 18 ml of DMF is stirred at RT for 3 days. The reaction mixture is poured onto ice. The solid which precipitates is collected by filtration, is washed with water and is air-dried. It is stirred in 50 ml of chloroform for 2 hours and is filtered. The filtrate is evaporated to give glass

like material, which is triturated with ether to give the crude product. Purification by prep. TLC gives acetyloxymethyl 2-acetylnicotinate 4-(3-fluorophenyl)semicarbazone, m.p. 159-160° (compound 54, Table C).

**EXAMPLE 9**

Following the procedure of Example 8, chloromethyl butanoate, 1-chloroethyl propionat, 1-bromo-2-methyl-n-butyl acetate, methyl iodide, bromomethyl acetate, 1-chloroethyl propionate, chloromethyl butanoate and chloromethyl octanoate are reacted with the corresponding sodium salt to give compounds 55-62 and 80-86 in Table C.

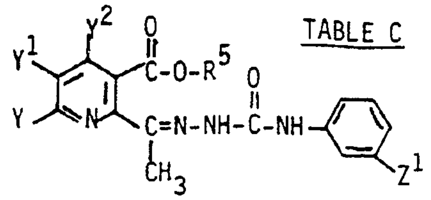

TABLE C

(Formula $A_5$)

| Cpd | Y | $Y^1$ | $Y^2$ | $R^5$ | $Z^1$ | m.p.° | Na salt m.p.° |
|---|---|---|---|---|---|---|---|
| 53 | H | H | H | $CH^3$ | H | 177-178 | - |
| 54 | H | H | H | $CH_2-O-\overset{O}{\overset{\|}{C}}-CH_3$ | F | 159-160 | - |
| 55 | H | H | H | $CH_2-O-\overset{O}{\overset{\|}{C}}-CH_2CH_2CH_3$ | F | 108-124 | - |
| 56 | H | H | H | $\overset{CH_3}{\overset{\|}{CH}}-O-\overset{O}{\overset{\|}{C}}-CH_2-CH_3$ | F | 158-164 | - |
| 57 | H | H | H | $\overset{CH_3CHCH_2CH_3}{\overset{\|}{CH}} - O - \overset{O}{\overset{\|}{C}} - CH_3$ | F | 182-186 | - |
| 58 | H | H | H | $CH_3$ | F | 206-214 | - |
| 59 | H | H | H | $CH_2-O-\overset{O}{\overset{\|}{C}}-CH_3$ | Cl | 148-150 | - |
| 60 | H | H | H | $\overset{CH_3}{\overset{\|}{CH}}-O-\overset{O}{\overset{\|}{C}}-CH_2CH_3$ | Cl | 168-170 | |
| 61 | H | H | H | $CH_2-O-\overset{O}{\overset{\|}{C}}-CH_2CH_2CH_3$ | Cl | 150-152 | - |
| 62 | H | H | H | $CH_2-O-\overset{O}{\overset{\|}{C}}-(CH_2)_6CH_3$ | Cl | 46-47 | - |
| 63 | $OCH_3$ | H | H | H | F | 220-240 | 208* |
| 64 | Cl | H | H | H | H | 225 | |
| 65 | Cl | H | H | H | F | 210-214* | $200^+$ |
| 66 | H | $OCH_2CH_3$ | H | H | H | 141-142 | |
| 67 | H | $OCH_2CH_3$ | H | H | F | 135-136 | 233-235 |

TABLE C(cont)

| Cpd | Y | $Y^1$ | $Y^2$ | $R^5$ | $Z^1$ | m.p.° | Na salt m.p.° |
|---|---|---|---|---|---|---|---|
| 68 | H | Cl | H | H | Cl | 163-164 | >330 |
| 69 | H | Cl | H | H | F | 157-159 | >320 |
| 70 | H | $C_6H_5$ | H | H | H | 193-195 | 242-244$^+$ |
| 71 | H | $C_6H_5$ | H | H | F | 240-250 | 246-248$^+$ |
| 72 | H | H | Cl | H | H | | |
| 73 | H | H | Cl | H | F | 214-215$^+$ | >320 |
| 74 | H | $C_2H_5$ | H | H | F | 178* | 190-193* |
| 75 | H | $CH_3$ | H | H | F | 167-169* | 228-230* |
| 76 | $CH_3$ | H | H | H | F | | |
| 77 | $C_2H_5$ | H | H | H | F | | |
| 78 | H | H | $CH_3$ | H | F | | |
| 79 | H | H | $C_2H_5$ | H | F | | |
| 80 | $OCH_3$ | H | H | $CH_2-O-\overset{O}{\overset{\Vert}{C}}-CH_2CH_2CH_3$ | F | | - |
| 81 | Cl | H | H | $CH_2-O-\overset{O}{\overset{\Vert}{C}}-CH_2CH_2CH_3$ | F | 118-120 | - |
| 82 | H | $OCH_2CH_3$ | H | $CH_2-O-\overset{O}{\overset{\Vert}{C}}-CH_2CH_2CH_3$ | F | 150-152 | - |
| 83 | H | Cl | H | $CH_2-O-\overset{O}{\overset{\Vert}{C}}-CH_2CH_3CH_3$ | F | 160-163 | - |
| 84 | H | $C_6H_5$ | H | $CH_2-O-\overset{O}{\overset{\Vert}{C}}-CH_2CH_2CH_3$ | F | 197-198 | - |
| 85 | H | H | H | $CH_2-O-CO-C(CH_3)_3$ | F | 173-175° | - |
| 86 | H | H | H | $CH_2-O-CO-nC_3H_7$ | $C_2H_5$ | 103-105° | - |

TABLE D

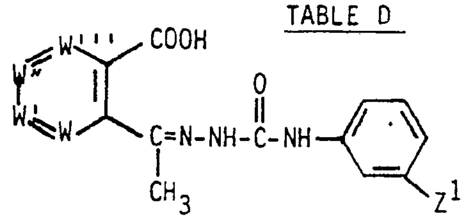

(Formula $A_6$)

| Cpd. No. | W | W' | W'' | W''' | $Z^1$ | m.p.° acid | m.p.° Na salt |
|---|---|---|---|---|---|---|---|
| 87 | CH | CH | CH | N | H | 160-162$^+$ | |
| 88 | CH | CH | CH | N | F | 125-133* | 198-199$^+$ |
| 89 | CH | N | CH | CH | H | 230-245 | |
| 90 | CH | N | CH | CH | F | | |
| 91 | CH | CH | N | CH | H | 180-182$^+$ | |
| 92 | CH | CH | N | CH | F | | |
| 93 | CH | N | C-Cl | N | H | 165-167 | 230-240* |
| 94 | CH | N | C-Cl | N | F | | |
| 95 | CH | N | CH | N | H | 194-195* | 214-216* |
| 96 | CH | N | CH | N | F | | |
| 97 | N | CH | N | CH | H | 196-198* | 207-208$^+$ |
| 98 | N | CH | N | CH | F | | |
| 99 | N | CH | CH | N | H | 164-169$^+$ | |
| 100 | N | CH | CH | N | F | | |

**EXAMPLE 10**

Following the procedure of Example 2 an acetyl-quinoline carboxylic acid is reacted with a semicabazide to give
2-acetyl-3-quinolinecarboxylic acid 4-phenylsemicarbazone, m.p. 234-236° (compound 101), its Na-salt having a m.p. of 224-225° (gas)
3-acetyl-2-quinolincarboxylic acid 4-phenylsemicarbazone, m.p. 243° (gas) (compound 102), its Na-salt having a m.p. of 300° ;
3-acetyl-4-quinolinecarboxylic acid 4-phenylsemicarbazone, m.p. 251° (dec.) (compound 103), its Na-salt having a m.p. of 300° ;
3-acetyl-4-quinolinecarboxylic acid 4-(3-chlorophenyl)semicarbazone, m.p. 229° (compound 104), both its Na-salt and isopropylammonium salt having a m.p. of 300°.

**EXAMPLE 11**

To a stirred solution of 2-acetyl-3-thiophenecarboxylic acid (1.70 g, 10.0 mmol) in 15 ml of dry methanol is added, all at once, 4-phenylsemicarbazide (1.51 g, 10.0 mmol) in 25 ml of dry methanol. Stirring is continued for 18 hours, after which the precipitate is filtered off, washed with diethyl ether and dried to give 2-acetyl-3-thiophenecarboxylic acid 4-phenylsemicarbazone, m.p. 238° (compound 105, Table E).

Compounds 106-125 (Table E) are obtained following the same procedure.

TABLE E

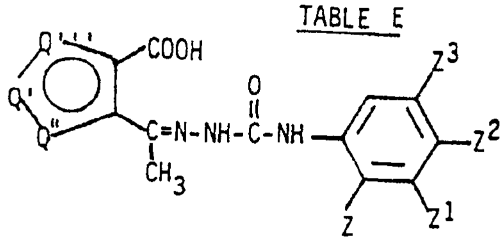

(Formula $A_7$)

| Cpd. No. | Q' | Q'' | Q''' | Z | $Z^1$ | $Z^2$ | $Z^3$ | m.p.° acid | m.p.° Na salt |
|---|---|---|---|---|---|---|---|---|---|
| 105 | CH | S | CH | H | H | H | H | 238 | 270 |
| 106 | CH | S | CH | Cl | H | H | H | 235 | >290 |
| 107 | CH | S | CH | H | Cl | H | H | 258 | |
| 108 | CH | S | CH | H | H | Cl | H | 240 | 203 |
| 109 | CH | S | CH | H | Cl | H | Cl | 210 | 240-244* |
| 110 | CH | S | CH | H | F | H | H | 252 | 222 |
| 111 | CH | S | CH | H | H | F | H | 260 | >280 |
| 112 | CH | S | CH | H | $OCH_3$ | H | H | 262 | >280 |
| 113 | CH | CH | S | H | H | H | H | 258 | 223 |
| 114 | CH | CH | S | Cl | H | H | H | 256 | 201-209* |
| 115 | CH | CH | S | H | Cl | H | H | 249 | 192-203 |
| 116 | CH | CH | S | H | H | Cl | H | 251 | 242 |
| 117 | CH | CH | S | H | Cl | H | Cl | 216 | 242-246 |
| 118 | CH | CH | S | H | F | H | H | 255 | 239-242 |
| 119 | CH | CH | S | H | H | F | H | 261 | 235-239 |
| 120 | CH | CH | S | H | $OCH_3$ | H | H | 253 | 225 |
| 121 | S | CH | CH | H | H | H | H | 170-171 | 232-233 |
| 122 | S | CH | CH | H | Cl | H | H | 174-175 | 223-224 |
| 123 | S | CH | CH | H | Cl | H | Cl | 207-208 | $210^+$ |
| 124 | S | CH | CH | H | F | H | H | 174-176 | $218-220^+$ |
| 125 | S | CH | CH | H | F | H | F | 194-195 | 227-229 |

TABLE E (cont.)

| Cpd. No. | Q' | Q'' | Q''' | Z | $Z^1$ | $Z^2$ | $Z^3$ | m.p.° acid | m.p.° Na salt |
|---|---|---|---|---|---|---|---|---|---|
| 126 | CH | O | CH | H | H | H | H | 240 | 210$^+$ |
| 127 | CH | O | CH | H | Cl | H | H | 256 | 235$^+$ |
| 128 | CH | O | CH | H | Cl | H | Cl | | |
| 129 | CH | O | CH | H | F | H | H | 253 | 225$^+$ |
| 130 | CH | O | CH | H | F | H | F | | |
| 131 | CH | CH | O | H | H | H | H | | |
| 132 | CH | CH | O | H | Cl | H | H | | |
| 133 | CH | CH | O | H | Cl | H | Cl | | |
| 134 | CH | CH | O | H | F | H | H | | |
| 135 | CH | CH | O | H | F | H | F | | |
| 136 | O | CH | CH | H | H | H | H | | |
| 137 | O | CH | CH | H | Cl | H | H | | |
| 138 | O | CH | CH | H | Cl | H | Cl | | |
| 139 | O | CH | CH | H | F | H | H | | |
| 140 | O | CH | CH | H | F | H | F | | |

EXAMPLE 12

To a suspension of 2-acetyl-3-nicotinic acid N-oxide (2.5 g, 13.8 mmol) in 40 ml of ethanol is added 4-phenylsemicarbazide (2.1 g, 13.8 mmol). The mixture is stirred at 40° overnight, after which the solvent is removed and the product purified to give 2-acetyl-3-nicotinic acid N-oxide 4-phenylsemicarbazone, m.p. 218° (dec.) (compound 141) ; its Na-salt melts at 128-133°, its $NH_4$-salt softens at 137° and enters the gas phase at 155°.

EXAMPLE 13

Following the procedure of Example 12 2-acetyl-3-nicotinic acid N-oxide 2-methyl-4-phenylsemicarbazone, m.p. 55° (softens), 65° (melts) (compound 142) is obtained.

EXAMPLE 14

A mixture of N,N-diethyl 2-acetyl-3-nicotinamide (0.45 g, 2.0 mmol) and 4-phenylsemicarbazide (0.31 g, 2.0 mmol) in 10 ml of acetic acid is stirred at RT overnight. The solvent is then removed by rotoevaporation, and the residue is triturated with methanol. The solid is collected by filtration and is dried to give N,N-diethyl-2-acetyl-3-nicotinamide 4-phenylsemicarbazone, m.p. 241-243° (compound 143).

EXAMPLE 15

Following the procedures of Example 14, each of 4-(3-fluorophenyl)semicarbazide and 4-(3-chlorophenyl)semicarbazide is reacted with N,N-diethyl 2-acetyl-3-nicotinamide to give N,N-diethyl 2-acetyl-3-nicotinamide 4-(3-fluorophenyl)semicarbazone (compound 144) and N,N-diethyl 2-acetyl-3-nicotinamide 4-(3-chlorophenyl)semicarbazone (compound 145) resp.

EXAMPLE 16

A solution of ethyl 2-methylnicotinate (2.0 g, 12.12 mmol), selenium dioxide (20.0 g, 18.18 mmol) and dioxane (10 ml) is heated at 135° for 4 hours. The reaction mixture is filtered and the filtrate is concentrated and purified by prep. TLC to give ethyl 2-formylnicotinate.

Ethyl 2-formylnicotinate (1.0 g, 5.58 mmol) in 1 ml of ethanol is added to an aqueous solution of lithium hydroxide (0.28 g, 6.70 mmol) in 3 ml of water. The mixture is stirred at RT for 2 hours, then concentrated under vacuum at RT. The resulting lithium salt of 2-formylnicotinic acid is dissolved in 5 ml of methanol, and 4-methylsemicarbazide (0.77 g, 5.07 mmol) is added. The mixture is stirred at RT overnight. The resulting white precipitate is filtered and washed with methanol to give the lithium salt of 2-formylnicotinic acid 4-phenylsemicarbazone, m.p. 266° (compound 146).

EXAMPLE 17

To a mixture of 4-phenyl-3-thiosemicarbazone of 2-acetylnicotinic acid (1.0 g, 3.2 mmol) and potassium carbonate (0.88 g, 6.4 mmol) in 6 ml of dimethylformamide was added 0.4 ml (0.9 g, 6.4 mmol) of methyl iodide. The mixture was stirred overnight, and then poured into ether/$CHCl_3$ and water. The organic phase is separated and dried ($Na_2SO_4$). Removal of solvent gave the compound of formula $A_1$ wherein R is 3-methoxycarbonyl-2-pyridyl, $R^3$ is $CH_3$, $R^2$ is C(=$NC_6$H5)-$SCH_3$ and $R^4$ is H (Compound 147) as a mixture of isomers (the isomer having the corresponding structure in which $NR^2R^4$ is N=C($SCH_3$)-NH-$C_6H_5$), which mixture was crystallized from $CH_3CN$.

EXAMPLE 18

0.64 g (1.9 mmol) of the compound of Example 17 (compound 147) was hydrolysed by stirring with lithium hydroxide monohydrate (0.08 g, 1.9 mmol) in 10 ml of methanol and 2 ml of water at 80° for six hours. The methanol and water were removed by rotoevaporation to give the compound of formula $A_1$ wherein R is 3-CO-OLi-2-pyridyl, $R^3$ is $CH_3$, $R^2$ is C(=$NC_6H_5$)-$SCH_3$ and $R^4$ is H (compound 148).

## EXAMPLE 19

0.6 g (1.9 mmol) of compound 148 was suspended in 5% HCl (8 ml), and the solution was separately extracted with $CHCl_3$. The chloroform extracts were dried ($Na_2SO_4$), and the chloroform was removed to give the corresponding acid of formula $A_1$, wherein R is 3-COOH-2-pyridyl, $R^3$ is $CH_3$, $R^2$ is C(=$NC_6H_5$)-$SCH_3$ and $R^4$ is H (compound 149) ; m.p. 78° (softens), 132-35° (gas).

## EXAMPLE 20

Pre-emergent herbicidal activity of selected compounds of the present invention was determined as follows : Seeds of selected weeds were planted and the soil was drenched with a solution of water (17%), surfactant (0.17%) and the test compound at a rate equivalent to 10 lb/acre. Scoring was made two weeks after treatment. The grasses (GR) Setaria viridis, Echinochloa crus-galli, Sorghum bicolor and Avena fatua and the broadleafs (BL) Ipomoea purpurea, Brassica juncea, Solanum nigrum and Abutilon theophrasti were treated. The average pre-emergent activity of the compounds is presented in Table F below (10 lb/acre corresponds roughly to 11 kg/ha).

## EXAMPLE 21

Post-emergence herbicidal activity of selected compounds of the present invention was tested as follows: Seedlings of selected weeds were sprayed with a solution of water/acetone (1 : 1), surfactant (0.5%) and the test compound at a rate equivalent to 10 lb/acre. Scoring was made two weeks after spraying. The grasses (GR) Setaria viridis, Echinochloa crusgalli, Sorghum bicolor and Avena fatua and the broadleafs (BL) Ipomoea purpurea, Brassica juncea, soybean and Abutilon theophrasti were treated. The average post-emergent activities of the compounds are presented in Table F below.

TABLE F

% Herbicidal Activity at 10 lb/Acre

| Compound No. | Pre GR | Pre BL | Post GR | Post BL |
|---|---|---|---|---|
| 1 | 91 | 86 | 48 | 61 |
| Na salt of 1 | 88 | 93 | 47 | 62 |
| 3 | 96 | 90 | 58 | 75 |
| Na salt of 3 | 93 | 89 | 63 | 81 |
| 4 | 91 | 91 | 72 | 85 |
| Na salt of 4 | 94 | 89 | 81 | 85 |
| $NH_4$ salt of 4 | 92 | 92 | 77 | 78 |
| isoprop $NH_3$ salt of 4 | 77 | 80 | 61 | 77 |
| diisoprop $NH_2$ salt of 4 | 82 | 80 | 63 | 80 |
| 22 | 58 | 58 | 30 | 71 |
| Na salt of 22 | 53 | 73 | 20 | 56 |
| 23 | 75 | 63 | 53 | 70 |
| Na salt of 23 | 68 | 80 | 66 | 73 |
| 24 | 92 | 86 | 55 | 76 |
| Na salt of 24 | 85 | 82 | 63 | 75 |
| 25 | 90 | 68 | 68 | 81 |
| Na salt of 25 | 79 | 90 | 66 | 85 |
| 30 | 96 | 87 | 78 | 86 |
| Na salt of 30 | 100 | 89 | 83 | 90 |

TABLE F (continued)

| Compound No. | Pre GR | Pre BL | Post GR | Post BL |
|---|---|---|---|---|
| 31 | 87 | 83 | 80 | 87 |
| Na salt of 31 | 96 | 100 | 71 | 86 |
| 32 | 92 | 95 | 90 | 95 |
| Na salt of 32 | 96 | 94 | 86 | 90 |
| 55 | 80 | 80 | 76 | 82 |
| 57 | 85 | 90 | 63 | 85 |
| 60 | 85 | 80 | 71 | 78 |
| 61 | 82 | 81 | 67 | 81 |
| 110 | 88 | 76 | -- | -- |
| Na salt of 110 | 81 | 80 | 60 | 80 |

## Claims

### Claims for the Contracting States : BE CH DE FR GB IT LI LU NL SE

1. Compounds of formula (A) :

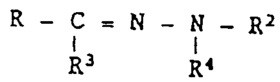

(A)

wherein R is phenyl or naphthyl, or is a heteroaromatic ring selected from

a) pyridyl which may be fused by its [b] or [c]-side with a benzene ring,

b) 2-pyridyl-N-oxide or 2-pyrazinyl-$N^1$-oxide,

c) pyrimidinyl,

d) pyrazinyl,

e) 3- or 4-cinnolinyl or 2-quinoxalinyl and

f) a five-membered heteroaromatic ring bound by one of its ring C-atoms to the $CR^3$-group selected from thienyl, furanyl, pyrrolyl, oxazolyl, thiazolyl, isoxazolyl, isothiazolyl, imidazolyl or pyrazolyl ; whereby said five-membered heteroaromatic ring may be fused by its [b]-side with a benzene ring ;

which group R isortho-substituted by the group R′ and additionally bears one further substituent Y, both R′ and Y being attached to a ring carbon atom ;

R′ is the carboxy group in free form, salt form or ester form or is the group $CO-SR^6$ or $CO-NR^7R^8$,

Y is H, $C_{1-5}$alkyl, $C_{1-8}$halloalkyl, $C_{1-8}$alkoxy, $C_{1-8}$haloalkoxy, $C_{2-8}$alkenyloxy, $C_{2-8}$haloalkenyloxy, $C_{2-8}$alkynyloxy, phenyl, phenoxy, $C_{1-8}$alkylthio, OH, halogen, nitro or cyano ;

$R^2$ is the group $-C(=NR^{10})-SR^9$ or $-C(=X)-NHR^{10}$, each of $R^3$, $R^4$, $R^7$ and $R^8$ is independently hydrogen or $C_{1-8}$alkyl ; each of $R^6$ and $R^9$ is independently $C_{1-8}$alkyl, $R^{10}$ is one of the groups

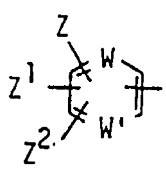

(G-1) ,

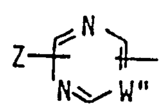

(G-2) ,

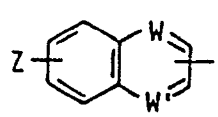

(G-3) or

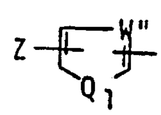

(G-4)

each of W, W′ and W″ is independently N or CH

$Q_1$ is oxygen, sulfur or NH

X is oxygen or sulfur each of Z, $Z^1$ and $Z^2$ is independently one of the significances specified for – but independent of – Y,

with the proviso that when R is phenyl mono-substituted in ortho by the carboxy group in free form or in salt form or by $C_{1-4}$alkoxy-carbonyl, then $R^3$ is $C_{1-8}$alkyl.

2. A compound of Claim 1, wherein R is 2-COOH-phenyl or 2-$COOR^5$phenyl, which phenyl group is substituted by Y, $R^2$ is a group $-C(=X)-NHR^{10}$, $R^3$ is $C_{1-5}$alkyl $R^4$ is H or $C_{1-5}$alkyl, R is $C_{1-5}$alkyl, $C_{1-5}$haloalkyl, $C_{2-10}$alkoxyalkyl or $-CH(-R^{11})-O-C(=O)-C_{1-8}$alkyl, $R^{10}$ is phenyl or pyridyl, which groups are substituted by Z and $Z^1$, $R^{11}$ is H or $C_{1-5}$alkyl, X is O or S, each of Y and Z is independently H, halogen, $C_{1-5}$alkyl, $C_{1-5}$alkoxy, $CF_3$, phenyl, phenoxy, nitro, cyano or hydroxy, and $Z^1$ is H, halogen, $C_{1-5}$alkyl or $C_{1-5}$alkoxy, whereby any carboxy group in R is in free form or in salt form.

3. A compound of claim 1, wherein R is a heteroaromatic ring selected from

a) pyridyl which may be fused by its [b] or [c]-side with a benzene ring,

b) 2-pyridyl-N-oxide or 2-pyrazinyl-$N^1$-oxide,

c) pyrimidinyl,

d) pyrazinyl,

e) 3- or 4-cinnolinyl or 2-quinoxalinyl and

f) a five-membered heteroaromatic ring bound by one of its ring C-atoms to the $CR^3$-group selected from thienyl, furanyl, pyrrolyl, oxazolyl, thiazolyl, isoxazolyl, isothiazolyl, imidazolyl or pyrazolyl ; whereby said

five-membered heteroaromatic ring may be fused by its [b]-side with a benzene ring ;

which group R is ortho-substituted by the group R′ and additionally bears one further substituent Y, both R′ and Y being attached to a ring carbon atom ;

R′ is the carboxy group in free form or salt form, or is the group $COOR^5$,

$R^3$ is H or $C_{1-5}$alkyl and $R^2$, $R^4$, $R^5$, $R^{10}$, $R^{11}$, X, Y, Z and $Z^1$ are as stated in Claim 2.

4. A compound of Claim 3, wherein the heteroaromatic ring R is selected from pyridyl, quinolyl, 2-pyridyl-N-oxide, pyrimidinyl, pyrazinyl, thienyl and furyl, substituted by R′ and Y as stated in Claim 3.

5. A compound of Claim 4, wherein R is selected from pyridyl or thienyl, substituted by R′ and Y as stated in Claim 3 and $R^4$ is H.

6. A compound of Claim 5, wherein $R^3$ is $C_{1-5}$alkyl, X is oxygen, $R^{10}$ is Z,$Z^1$-substituted phenyl, Z is H, halogen, $C_{1-5}$alkyl, $C_{1-5}$alkoxy or $CF_3$ and $Z^1$ is H, chloro, fluoro, methyl or methoxy.

7. A compound of Claim 6, wherein R is 2-pyridyl ortho-substituted by R′ as stated in Claim 3 and Y being H.

8. A compound of Claim 6, wherein R is thienyl ortho-substituted by R′ as stated in Claim 3 and Y being H.

9. A compound of Claim 7, wherein $R^{10}$ is phenyl, 3-chlorophenyl or 3-fluorophenyl.

10. A compound of any of Claims 1 to 9 wherein R′ is the carboxy group in free form or salt form or is a group $COO-CH(R^{11})-O-C(=O)-C_{1-5}$alkyl, wherein $R^{11}$ is as stated in Claim 2.

11. A compound of Claim 10 selected from

a) 2-acetylnicotinic acid 4-phenylsemicarbazone in free acid form or salt form,

b) 2-acetylnicotinic acid 4-(3-chlorophenyl)semicarbazone in free form or salt form,

c) 2-acetylnicotinic acid 4-(3-fluorophenyl)-semicarbazone in free form or salt form,

d) butyryloxymethyl 2-acetylnicotinate 4-(3-fluorophenyl)-semicarbazone,

e) butyryloxymethyl 2-acetylnicotinate 4-(3-chlorophenyl)-semicarbazone.

12. Process of preparing a compound of formula (A) stated in Claim 1, which comprises

a) reacting a compound of formula I

$$R\text{-}C(=O)R^3 \quad I$$

wherein R and $R^3$ are as defined in Claim 1, with a compound of formula II

$$H_2N\text{-}NR^2R^4 \quad II$$

wherein $R^2$ and $R^4$ are as defined in Claim 1, followed, where desired, by esterifying compounds of formula (A) wherein R′ is the carboxyl group to compounds (A) wherein R′ is a carboxyl-ester group, or

b) S-alkylating a compound of formula $A_1$

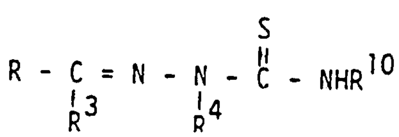

$(A_1)$

wherein R, $R^3$, $R^4$ and $R^{10}$ are as defined in Claim 1, with an alkylhalogenide of formula III

$$R^9Hal \quad III$$

wherein $R^9$ is as defined in Claim 1 and Hal is halogen, or a reactive functional derivative of said compound of formula III to give a compound of formula $(A_2)$

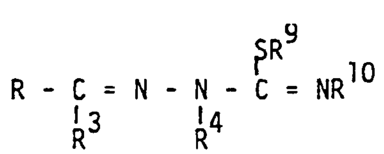

$(A_2)$

wherein R, $R^3$, $R^4$, $R^9$ and $R^{10}$ are as defined in Claim 1.

13. A herbicidal composition comprising a compound of formula (A) according to any one of Claims 1 to

11 and an agriculturally acceptable diluent.

14. A method of combatting weeds which comprises applying to the locus thereof a herbicidally effective amount of a compound of formula (A) according to any one of Claims 1 to 11.

**Claims for the Contracting State : AT**

1. A herbicidal composition comprising a compound of formula (A) :

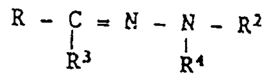 (A)

wherein R is phenyl or naphthyl, or is a heteroaromatic ring selected from

a) pyridyl which may be fused by its [b] or [c]-side with a benzene ring,

b) 2-pyridyl-N-oxide or 2-pyrazinyl-$N^1$-oxide,

c) pyrimidinyl,

d) pyrazinyl,

e) 3- or 4-cinnolinyl or 2-quinoxalinyl and

f) a five-membered heteroaromatic ring bound by one of its ring C-atoms to the $CR^3$-group selected from thienyl, furanyl, pyrrolyl, oxazolyl, thiazolyl, isoxazolyl, isothiazolyl, imidazolyl or pyrazolyl ; whereby said five-membered heteroaromatic ring may be fused by its [b]-side with a benzene ring ;

which group R is ortho-substituted by the group R′ and additionally bears one further substituent Y, both R′ and Y being attached to a ring carbon atom ;

R′ is the carboxy group in free form, salt form or ester form or is the group $CO\text{-}SR^6$ or $CO\text{-}NR^7R^8$,

Y is H, $C_{1-5}$alkyl, $C_{1-8}$haloalkyl, $C_{1-8}$alkoxy, $C_{1-8}$haloalkoxy, $C_{2-8}$alkenyloxy, $C_{2-8}$haloalkenyloxy, $C_{2-8}$alkynyloxy, phenyl, phenoxy, $C_{1-8}$alkylthio, OH, halogen, nitro or cyano ;

$R^2$ is the group $-C(=NR^{10})\text{-}SR^9$ or $-C(=X)\text{-}NHR^{10}$, each of $R^3$, $R^4$, $R^7$ and $R^8$ is independently hydrogen or $C_{1-8}$alkyl ; each of $R^6$ and $R^9$ is independendly $C_{1-8}$alkyl,

$R^{10}$ is one of the groups

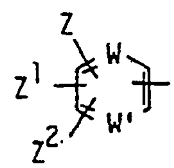
(G-1) ,
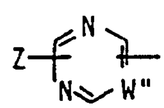
(G-2) ,
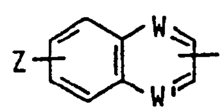
(G-3) or
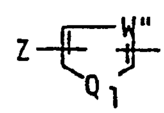
(G-4)

each of W, W′ and W″ is independently N or CH

$Q_1$ is oxygen, sulfur or NH

X is oxygen or sulfur each of Z, $Z^1$ and $Z^2$ is independently one of the significances specified for – but independent of – Y,

with the proviso that when R is phenyl mono-substituted in ortho by the carboxy group in free form or in salt form or by $C_{1-4}$alkoxy-carbonyl, then $R^3$ is $C_{1-4}$alkyl.

2. A composition of Claim 1, wherein R is 2-COOH-phenyl, or 2-$COOR^5$-phenyl which phenyl group is substituted by Y, $R^2$ is a group $-C(=X)\text{-}NHR^{10}$, $R^3$ is $C_{1-5}$-alkyl $R^4$ is H or $C_{1-5}$alkyl, $R^5$ is $C_{1-5}$alkyl, $C_{1-5}$haloalkyl, $C_{2-10}$alkoxyalkyl or $-CH(-R^{11})\text{-}O\text{-}C(=O)\text{-}C_{1-8}$alkyl, $R^{10}$ is phenyl or pyridyl, which groups are substituted by Z and $Z^1$, $R^{11}$ is H or $C_{1-5}$alkyl, X is O or S, each of Y and Z is independently H, halogen, $C_{1-5}$alkyl, $C_{1-5}$alkoxy $CF_3$, phenyl, phenoxy, nitro, cyano or hydroxy, and $Z^1$ is H, halogen, $C_{1-5}$alkyl or $C_{1-5}$alkoxy, whereby any carboxy group in R is in free form or in salt form.

3. A composition of Claim 1, wherein R is a heteroaromatic ring selected from

a) pyridyl which may be fused by its [b] or [c]-side with a benzene ring,

b) 2-pyridyl-N-oxide or 2-pyrazinyl-$N^1$-oxide,

c) pyrimidinyl,

d) pyrazinyl,

e) 3- or 4-cinnolinyl or 2-quinoxalinyl and

f) a five-membered heteroaromatic ring bound by one of its ring C-atoms to the $CR^3$-group selected from thienyl, furanyl, pyrrolyl, oxazolyl, thiazolyl, isoxazolyl, isothiazolyl, imidazolyl or pyrazolyl ; whereby said

five-membered heteroaromatic ring may be fused by its [b]-side with a benzene ring ;

which group R is ortho-substituted by the group R′ and additionally bears one further substituent Y, both R′ and Y being attached to a ring carbon atom ;

R′ is the carboxy group in free form or salt form, or is the group $COOR^5$,

$R^3$ is H or $C_{1-5}$alkyl and $R^2$, $R^4$, $R^5$, $R^{10}$, $R^{11}$, X, Y, Z and $Z^1$ are as stated in Claim 2.

4. A composition of Claim 3, wherein the heteroaromatic ring R is selected from pyridyl, quinolyl, 2-pyridyl-N-oxide, pyrimidinyl, pyrazinyl, thienyl and furyl, substituted by R′ and Y as stated in Claim 3.

5. A composition of Claim 4, wherein R is selected from pyridyl or thienyl, substituted by R′ and Y as stated in Claim 3 and $R^4$ is H.

6. A composition of Claim 5, wherein $R^3$ is $C_{1-5}$alkyl, X is oxygen, $R^{10}$ is Z,$Z^1$-substituted phenyl, Z is H, halogen, $C_{1-5}$alkyl, $C_{1-5}$alkoxy or $CF_3$ and $Z^1$ is H, chloro, fluoro, methyl or methoxy.

7. A composition of Claim 6, wherein R is 2-pyridyl ortho-substituted by R′ as stated in Claim 3 and Y being H.

8. A composition of Claim 6, wherein R is thienyl ortho-substituted by R′ as stated in Claim 3 and Y being H.

9. A composition of Claim 7, wherein $R^{10}$ is phenyl, 3-chlorophenyl or 3-fluorophenyl.

10. A composition of any of Claims 1 to 9 wherein R′ is the carboxy group in free form or salt form or is a group COO-CH($R^{11}$)-O-C(=O)-$C_{1-5}$alkyl, wherein $R^{11}$ is as stated in Claim 2.

11. A composition of Claim 10 comprising a compound selected from

a) 2-acetylnicotinic acid 4-phenylsemicarbazone in free acid form or salt form,

b) 2-acetylnicotinic acid 4-(3-chlorophenyl)semicarbazone in free form or salt form,

c) 2-acetylnicotinic acid 4-(3-fluorophenyl)-semicarbazone in free form or salt form,

d) butyryloxymethyl 2-acetylnicotinate 4-(3-fluorophenyl)-semicarbazone,

e) butyryloxymethyl 2-acetylnicotinate 4-(3-chlorophenyl)-semicarbazone.

12. Process of preparing a compound of formula (A) stated in Claim 1, which comprises

a) reacting a compound of formula I

$$R\text{-}C(=O)\text{-}R^3 \quad I$$

wherein R and $R^3$ are as defined in Claim 1, with a compound of formula II

$$H_2N\text{-}NR^2R^4 \quad II$$

wherein $R^2$ and $R^4$ are as defined in Claim 1, followed, where desired, by esterifying compounds of formula (A) wherein R′ is the carboxyl group to compounds (A) wherein R′ is a carboxyl-ester group, or

b) S-alkylating a compound of formula $A_1$

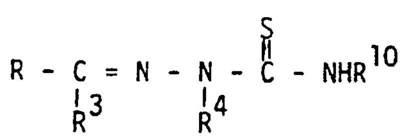

$(A_1)$

wherein R, $R^3$, $R^4$ and $R^{10}$ are as defined in Claim 1, with an alkylhalogenide of formula III

$$R^9Hal \quad III$$

wherein $R^9$ is as defined in Claim 1 and Hal is halogen, or a reactive functional derivative of said compound of formula III to give a compound of formula $(A_2)$

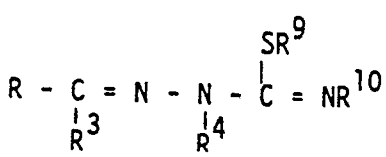

$(A_2)$

wherein R, $R^3$, $R^4$, $R^9$ and $R^{10}$ are as defined in Claim 1.

**Ansprüche**

**Patentansprüche für die Vertragsstaaten : BE, CH/LI, DE, FR, GB, IT, LU, NL, SE**

1. Verbindungen der Formel (A) :

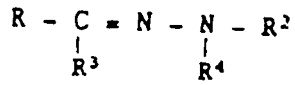 (A)

worin R eine Phenyl- oder Naphthylgruppe ist oder ein heteroaromatischer Ring, ausgewählt aus

a) einer Pyridylgruppe, die an ihrer [b]- oder [c]-Seite an einen Benzolring kondensiert sein kann,
b) 2-Pyridyl-N-oxid oder 2-Pyrazinyl-$N^1$-oxid,
c) einer Pyrimidinylgruppe,
d) einer Pyrazinylgruppe,
e) einer 3- oder 4-Cinnolinyl- oder 2-Chinoxalinylgruppe und
f) einem fünfgliedrigen heteroaromatischen Ring, der mit einem seiner Ring-C-Atome an die $CR^3$-Gruppe, ausgewählt aus Thienyl, Furanyl, Pyrrolyl, Oxazolyl, Thiazolyl, Isoxazolyl, Isothiazolyl, Imidazolyl oder Pyrazolyl, gebunden ist ; wobei dieser fünfgliedrige heteroaromatische Ring an seiner [b]-Seite an einen Benzolring kondensiert sein kann ;

wobei die Gruppe R durch die Gruppe R′ ortho-substituiert ist und zusätzlich einen weiteren Substituenten Y trägt, und sowohl R′ als auch Y an ein Ringkohlenstoffatom gebunden sind ;

R′ die Carboxygruppe in freier Form, Salzform oder Esterform ist oder die Gruppe $CO\text{-}SR^6$ oder $CO\text{-}NR^7R^8$ ist,

Y H, eine $C_{1-5}$-Alkyl-, $C_{1-8}$-Halogenalkyl-, $C_{1-8}$-Akoxy-, $C_{1-8}$-Halogenalkoxy-, $C_{2-8}$-Alkenyloxy-, $C_{2-8}$-Halogenalkenyloxy-, $C_{2-8}$-Alkynyloxy-, Phenyl-, Phenoxy-, $C_{1-8}$-Alkylthio-, OH-Gruppe, Halogen, eine Nitro- oder Cyanogruppe ist ;

$R^2$ die Gruppe $-C(=NR^{10})-SR^9$ oder $-C(=X)-NHR^{10}$ ist, wobei $R^3$, $R^4$, $R^7$ und $R^8$ jeweils unabhängig Wasserstoff oder eine $C_{1-8}$-Alkylgruppe ist ; $R^6$ und $R^9$ jeweils unabhängig eine $C_{1-8}$-Alkylgruppe ist, $R^{10}$ eine der Gruppen

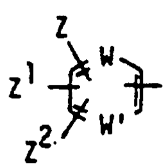 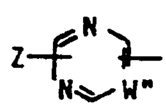 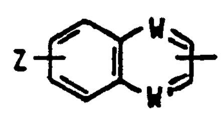 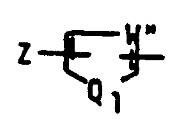

(G-1) , (G-2) , (G-3) oder (G-4)

ist, W, W′ und W″ jeweils unabhängig N oder CH ist, $Q_1$ Sauerstoff, Schwefel oder NH ist, X Sauerstoff oder Schwefel ist, Z, $Z^1$ und $Z^2$ jeweils unabhängig eine der für Y angegebenen – aber unabhängig von Y – Bedeutungen hat, mit dem Vorbehalt, daß dann, wenn R eine in ortho-Stellung durch die Carboxygruppe in freier Form oder in Salzform oder mit der $C_{1-4}$-Alkoxycarbonylgruppe monosubstituierte Phenylgruppe ist, daß dann $R^3$ eine $C_{1-8}$-Alkylgruppe ist.

2. Verbindung nach Anspruch 1, worin R eine 2-COOH-Phenyl- oder 2-$COOR^5$-Phenylgruppe ist, wobei die Phenylgruppe mit Y substituiert ist, $R^2$ eine Gruppe $-C(=X)-NHR^{10}$ ist, $R^3$ eine $C_{1-5}$-Alkylgruppe ist, $R^4$ H oder eine $C_{1-5}$-Alkylgruppe ist, $R^5$ eine $C_{1-5}$-Alkyl-, $C_{1-5}$-Halogenalkyl-, $C_{2-10}$-Alkoxyalkyl- oder $-CH(-R^{11})-O-C(=O)-C_{1-8}$-Alkylgruppe ist, $R^{10}$ eine Phenyl- oder Pyridylgruppe ist, wobei diese Gruppen mit Z und $Z^1$ substituiert sind, $R^{11}$ H oder eine $C_{1-5}$-Alkylgruppe ist, X O oder S ist, Y und Z jeweils unabhängig H, Halogen, eine $C_{1-5}$-Alkyl-, $C_{1-5}$-Akoxy-, $CF_3$-, Phenyl-, Phenoxy-, Nitro-, Cyano- oder Hydroxygruppe ist und $Z^1$ H, Halogen, eine $C_{1-5}$-Alkyl- oder $C_{1-5}$-Alkoxygruppe ist, wobei jede Carboxygruppe in R in freier oder in Salzform vorliegt.

3. Verbindung nach Anspruch 1, worin R ein heteroaromatischer Ring ist, ausgewählt aus

a) einer Pyridylgruppe, die an ihrer[b]- oder [c]-Seite an einen Benzolring kondensiert sein kann,
b) 2-Pyridyl-N-oxid oder 2-Pyrazinyl-$N^1$-oxid,
c) einer Pyrimidinylgruppe,
d) einer Pyrazinylgruppe,
e) einer 3- oder 4-Cinnolinyl- oder 2-Chinoxalinylgruppe und
f) einem fünfgliedrigen heteroaromatischen Ring, der mit einem seiner Ring-C-Atome an die $CR^3$-Gruppe,

ausgewählt aus Thienyl, Furanyl, Pyrrolyl, Oxazolyl, Thiazolyl, Isoxazolyl, Isothiazolyl, Imidazolyl oder Pyrazolyl, gebunden ist ; wobei der fünfgliedrige heteroaromatische Ring an seiner[b]-Seite an einen Benzolring kondensiert sein kann ;

wobei die Gruppe R ortho-substituiert ist mit der Gruppe R′ und zusätzlich einen weiteren Substituenten Y trägt, und sowhol R′ als auch Y an ein Ringkohlenstoffatom gebunden sind ; R′ die Carboxygruppe in freier Form oder Salzform ist oder die Gruppe $COOR^5$ ist, $R^3$ H oder eine $C_{1-5}$-Alkylgruppe ist und $R^2$, $R^4$, $R^5$, $R^{10}$, $R^{11}$, X, Y, Z und $Z^1$ wie in Anspruch 2 angegeben sind.

4. Verbindung nach Anspruch 3, worin der heteroaromatische Ring R ausgewählt ist aus Pyridyl, Chinolyl, 2-Pyridyl-N-oxid, Pyrimidinyl, Pyrazinyl, Thienyl und Furyl, substituiert mit R′ und Y, wie in Anspruch 3 angegeben.

5. Verbindung nach Anspruch 4, worin R ausgewählt ist aus Pyridyl oder Thienyl, substituiert mit R′ und Y, wie in Anspruch 3 angegeben, und $R^4$ H ist.

6. Verbindung nach Anspruch 5, worin $R^3$ eine $C_{1-5}$-Alkylgruppe ist, X Sauerstoff ist, $R^{10}$ eine Z,$Z^1$-substituierte Phenylgruppe ist, Z H, Halogen, eine $C_{1-5}$-Alkyl-, $C_{1-5}$-Alkoxy- oder $CF_3$-Gruppe ist und Z H, Chlor, Fluor, eine Methyl- oder Methoxygruppe ist.

7. Verbindung nach Anspruch 6, worin R ein mit wie in Anspruch 3 definiertem R′ ortho-substituierter 2-Pyridylrest ist und Y H ist.

8. Verbindung nach Anspruch 6, worin R ein mit wie in Anspruch 3 definiertem R′ ortho-substituierter Thienylrest ist und Y H ist.

9. Verbindung nach Anspruch 7, worin $R^{10}$ eine Phenyl-, 3-Chlorphenyl- oder 3-Fluorphenylgruppe ist.

10. Verbindung nach einem der Ansprüche 1 bis 9, worin R′ die Carboxygruppe in freier Form oder in Salzform ist oder eine Gruppe COO-CH($R^{11}$)-O-C(=O)-$C_{1-5}$-Alkylgruppe, worin $R^{11}$ wie in Anspruch 2 definiert ist.

11. Verbindung nach Anspruch 10, ausgewählt aus

a) 2-Acetylnicotinsäure-4-phenylsemicarbazon in freier Säure-form oder Salzform,

b) 2-Acetylnicotinsäure-4-(3-chlorphenyl)semicarbazon in freier Form oder in Salzform,

c) 2-Acetylnicotinsäure-4-(3-fluorphenyl)semicarbazon in freier Form oder in Salzform,

d) Butyryloxymethyl-2-acetylnicotinat-4-(3-fluorphenyl)semicarbazon,

e) Butyryloxymethyl-2-acetylnicotinat-4-(3-chlorphenyl)semicarbazon.

12. Verfahren zur Herstellung einer Verbindung der Formel (A), wie in Anspruch 1 definiert, das umfaßt, daß man

a) eine Verbindung der Formel I

$$R\text{-}C(=O)\text{-}R^3 \quad I,$$

worin R und $R^3$ wie in Anspruch 1 definiert sind, mit einer Verbindung der Formel II

$$H_2N\text{-}NR^2R^4 \quad II,$$

worin $R^2$ und $R^4$ wie in Anspruch 1 definiert sind, umsetzt und anschließend, falls erwünscht, die Verbindungen der Formel (A), worin R′ die Carboxylgruppe ist, zu Verbindungen (A) verestert, worin R′ eine Carboxylestergruppe ist, oder

b) eine Verbindung der Formel $A_1$

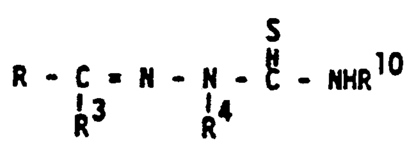

$(A_1)$

worin R, $R^3$, $R^4$ und $R^{10}$ wie in Anspruch 1 definiert sind, mit einem Alkylhalogenid der Formel III

$$R^9Hal \quad III,$$

worin $R^9$ wie in Anspruch 1 definiert ist und Hal Halogen ist, oder einem reaktiven funktionellen Derivat der Verbindung der Formel III S-alkyliert, um eine Verbindung der Formel ($A_2$)

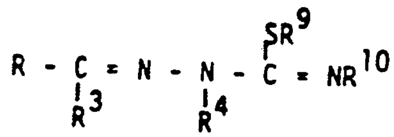

$(A_2)$

zu erhalten, worin R, $R^3$, $R^4$, $R^9$ und $R^{10}$ wie in Anspruch 1 definiert sind.

13. Herbicidzusammensetzung, umfassend eine Verbindung der Formel (A) gemäß einem der Ansprüche 1 bis 11 und ein landwirtschaftlich annehmbares Verdünnungsmittel.

14. Verfahren zur Bekämpfung von Unkraut, umfassend das Aufbringen einer herbicid wirksamen Menge einer Verbindung der Formel (A) gemäß einem der Ansprüche 1 bis 11 an seinem Standort.

**Patentansprüche für den Vertragsstaat : AT**

1. Herbicidzusammensetzung, umfassend eine Verbindung der Formel (A) :

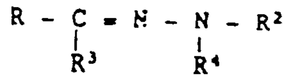

(A)

worin R eine Phenyl- oder Naphthylgruppe oder ein heteroaromatischer Ring, ausgewählt aus

a) einer Pyridylgruppe, die an ihrer [b]- oder [c]-Seite an einen Benzolring kondensiert sein kann,
b) 2-Pyridyl-N-oxid oder 2-Pyrazinyl-$N^1$-oxid,
c) einer Pyrimidinylgruppe,
d) einer Pyrazinylgruppe,
e) einer 3- oder 4-Cinnolinyl- oder 2-Chinoxalinylgruppe und
f) einem fünfgliedrigen heteroaromatischen Ring, der mit einem seiner Ring-C-Atome an die $CR^3$-Gruppe, ausgewählt aus Thienyl, Furanyl, Pyrrolyl, Oxazolyl, Thiazolyl, Isoxazolyl, Isothiazolyl, Imidazolyl oder Pyrazolyl gebunden ist ; wobei der fünfgliedrige heteroaromatische Ring an seiner [b]-Seite an einen Benzolring kondensiert sein kann ;

wobei die Gruppe R mit der Gruppe R′ in ortho-Stellung substituiert ist und zusätzlich einen weiteren Substituenten Y trägt und sowohl R′ als auch Y an ein Ringkohlenstoffatom gebunden sind ; R′ die Carboxygruppe in freier Form, Salzform oder Esterform oder die Gruppe $CO\text{-}SR^6$ oder $CO\text{-}NR^7R^8$ ist, Y H, eine $C_{1-5}$-Alkyl-, $C_{1-8}$-Halogenalkyl-, $C_{1-8}$-Alkoxy-, $C_{1-8}$Halogenalkoxy-, $C_{2-8}$-Alkenyloxy-, $C_{2-8}$-Halogenalkenyloxy-, $C_{2-8}$-Alkynyloxy-, Phenyl-, Phenoxy-, $C_{1-8}$-Alkylthio-, OH-Gruppe, Halogen, eine Nitro- oder Cyanogruppe ist ;

$R^2$ die Gruppe $-C(=NR^{10})\text{-}SR^9$ oder $-C(=X)\text{-}NHR^{10}$ ist, $R^3$, $R^4$, $R^7$ und $R^8$ jeweils unabhängig Wasserstoff oder eine $C_{1-8}$-Alkylgruppe ist ; $R^6$ und $R^9$ jeweils unabhängig eine $C_{1-8}$-Alkylgruppe ist, $R^{10}$ eine der Gruppen

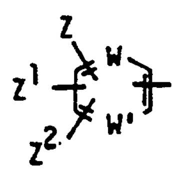

(G-1) ,

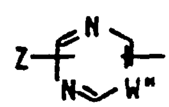

(G-2) ,

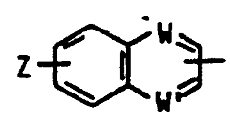

(G-3) oder

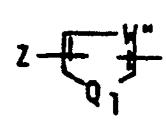

(G-4)

ist, W, W′ und W″ jeweils unabhängig N oder CH ist, $Q_1$ Sauerstoff, Schwefel oder NH ist, X Sauerstoff oder Schwefel ist, Z, $Z^1$ und $Z^2$ jeweils unabhängig eine der für Y angegebenen – aber unabhängig davon – Bedeutungen hat, mit dem Vorbehalt, daß dann, wenn R eine in ortho-Stellung mit der Carboxygruppe in freier Form oder in Salzform oder mit einer $C_{1-4}$-Alkoxycarbonylgruppe monosubstituierte Phenylgruppe ist, daß dann $R^3$ eine $C_{1-8}$-Alkylgruppe ist.

2. Zusammensetzung nach Anspruch 1, worin R eine 2-COOH-Phenyl- oder $2\text{-}COOR^5$-Phenylgruppe ist, wobei die Phenylgruppe mit Y substituiert ist, $R^2$ eine Gruppe $-C(=X)\text{-}NHR^{10}$, $R^3$ eine $C_{1-5}$-Alkylgruppe ist, $R^4$ H oder eine $C_{1-5}$-Alkylgruppe ist, $R^5$ eine $C_{1-5}$-Alkyl-, $C_{1-5}$-Halogenalkyl-, $C_{2-10}$-Alkoxyalkyl- oder $-CH(\text{-}R^{11})\text{-}O\text{-}C(=O)\text{-}C_{1-8}$-Alkylgruppe ist, $R^{10}$ eine Phenyl- oder Pyridylgruppe ist, wobei die Gruppen mit Z und $Z^1$ substituiert sind, $R^{11}$ H oder eine $C_{1-5}$-Alkylgruppe ist, X O oder S ist, Y und Z jeweils unabhängig H, Halogen, eine $C_{1-5}$-Alkyl-, $C_{1-5}$-Alkoxy-, $CF_3$-, Phenyl-, Phenoxy-, Nitro-, Cyano- oder Hydroxygruppe ist und $Z^1$ H, Halogen, eine

$C_{1-5}$-Alkyl- oder $C_{1-5}$-Alkoxygruppe ist, wobei jede Carboxygruppe in R in freier Form oder in Salzform vorliegt.

3. Zusammensetzung nach Anspruch 1, worin R ein heteroaromatischer Ring ist, ausgewählt aus

a) einer Pyridylgruppe, die an ihrer[b]- oder [c]-Seite an einen Benzolring kondensiert sein kann,

b) 2-Pyridyl-N-oxid oder 2-Pyrazinyl-$N^1$-oxid,

c) einer Pyrimidinylgruppe,

d) einer Pyrazinylgruppe,

e) einer 3- oder 4-Cinnolinyl- oder 2-Chinoxalinylgruppe und

f) einem fünfgliedrigen heteroaromatischen Ring, der mit einem seiner Ring-C-Atome an die $CR^3$-Gruppe, ausgewählt aus Thienyl, Furanyl, Pyrrolyl, Oxazolyl, Thiazolyl, Isoxazolyl, Isothiazolyl, Imidazolyl oder Pyrazolyl gebunden ist ; wobei der fünfgliedrige heteroaromatische Ring an seiner[b]-Seite an einen Benzolring kondensiert sein kann ;

wobei die Gruppe R mit der Gruppe R′ ortho-substituiert ist und zusätzlich einen weiteren Substituenten Y trägt und sowohl R′ als auch Y an ein Ringkohlenstoffatom gebunden sind ; R′ die Carboxygruppe in freier Form oder in Salzform oder die Gruppe $COOR^5$ ist, $R^3$ H oder eine $C_{1-5}$-Alkylgruppe ist und $R^2$, $R^4$, $R^5$, $R^{10}$, $R^{11}$, X, Y, Z und $Z^1$ wie in Anspruch 2 definiert sind.

4. Zusammensetzung nach Anspruch 3, worin der heteroaromatische Ring R ausgewählt ist aus Pyridyl, Chinolyl, 2-Pyridyl-N-oxid, Pyrimidinyl, Pyrazinyl, Thienyl und Furyl, substituiert mit R′ und Y wie in Anspruch 3 definiert.

5. Zusammensetzung nach Anspruch 4, worin R ausgewählt ist aus Pyridyl oder Thienyl, substituiert mit R′ und Y, wie in Anspruch 3 angegeben, und $R^4$ H ist.

6. Zusammensetzung nach Anspruch 5, worin $R^3$ eine $C_{1-5}$-Alkylgruppe ist, X Sauerstoff ist, $R^{10}$ eine $Z,Z^1$-substituierte Phenylgruppe ist, Z H, Halogen, eine $C_{1-5}$-Alkyl-, $C_{1-5}$-Alkoxy- oder $CF_3$-Gruppe ist und $Z^1$ H, Chlor, Fluor, eine Methyl- oder Methoxygruppe ist.

7. Zusammensetzung nach Anspruch 6, worin R ein mit wie in Anspruch 3 definiertem R′ ortho-substituierter 2-Pyridylrest ist und Y H ist.

8. Zusammensetzung nach Anspruch 6, worin R ein mit wie in Anspruch 3 definiertem R′ ortho-substituierter Thienylrest ist und Y H ist.

9. Zusammensetzung nach Anspruch 7, worin $R^{10}$ eine Phenyl-, 3-Chlorphenyloder 3-Fluorphenylgruppe ist.

10. Zusammensetzung nach einem der Ansprüche 1 bis 9, worin R′ die Carboxygruppe in freier Form oder in Salzform ist oder eine $COO-CH(R^{11})-O-C(=O)-C_{1-5}$-Alkylgruppe, worin $R^{11}$ wie in Anspruch 2 definiert ist.

11. Zusammensetzung nach Anspruch 10, umfassend eine Verbindung, ausgewählt aus

a) 2-Acetylnicotinsäure-4-phenylsemicarbazon in freier Säureform oder Salzform,

b) 2-Acetylnicotinsäure-4-(3-chlorphenyl)semicarbazon in freier Form oder in Salzform,

c) 2-Acetylnicotinsäure-4-(3-fluorphenyl)semicarbazon in freier Form oder in Salzform,

d) Butyryloxymethly-2-acetylnicotinat-4-(3-fluorphenyl)semicarbazon,

e) Butyryloxymethyl-2-acetylnicotinat-4-(3-chlorphenyl)semicarbazon.

12. Verfahren zur Herstellung einer Verbindung der Formel (A), wie in Anspruch 1 definiert, das umfaßt, daß man

a) eine Verbindung der Formel I

$$R-C(=O)-R^3 \quad I,$$

worin R und $R^3$ wie in Anspruch 1 definiert sind, mit einer Verbindung der Formel II

$$H_2N-NR^2R^4 \quad II,$$

worin $R^2$ und $R^4$ wie in Anspruch 1 definiert sind, umsetzt und anschließend, falls erwünscht, die Verbindungen der Formel (A), worin R′ die Carboxylgruppe ist, zu Verbindungen (A) verestert, worin R′ eine Carboxylestergruppe ist, oder

b) eine Verbindung der Formel $A_1$

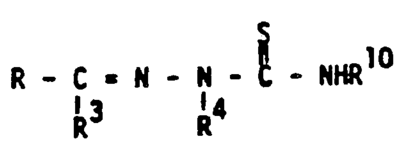

$(A_1)$

worin R, $R^3$, $R^4$ und $R^{10}$ wie in Anspruch 1 definiert sind, mit einem Alkylhalogenid der Formel III

$R^9$Hal III,

worin $R^9$ wie in Anspruch 1 definiert ist und Hal Halogen ist, oder einem reaktiven funktionellen Derivat der Verbindung der Formel III S-alkyliert, um eine Verbindung der Formel ($A_2$)

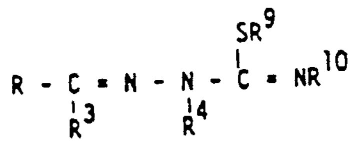

($A_2$)

zu erhalten, worin R, $R^3$, $R^4$, $R^9$ und $R^{10}$ wie in Anspruch 1 definiert sind.

## Revendications

### Revendications pour les Etats contractants : BE, CH, DE, FR, GB, IT, LI, LU, NL, SE.

1. Les composés de formule (A) :

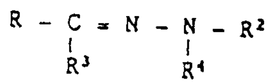

(A)

dans laquelle

R signifie phényle ou naphtyle ou un cycle hétéroaromatique choisi parmi les cycles

a) pyridyle qui peut être condensé par son côté [b] ou [c] avec un cycle benzénique,

b) 2-pyridyl-N-oxyde ou 2-pyrazinyl-$N^1$-oxyde,

c) pyrimidinyle,

d) pyrazinyle,

e) 3- ou 4-cinnolinyle ou 2-quinoxalinyle et

f) un cycle hétéroaromatique à 5 chaînons fixé par l'un de ses atomes de carbone du cycle au groupe $CR^3$-, choisi parmi les cycles thiényle, furannyle, pyrrolyle, oxazolyle, thiazolyle, isoxazolyle, isothiazolyle, imidazolyle et pyrazolyle, ledit cycle hétéroaromatique à 5 chaînons pouvant être condensé par son côté [b] avec un cycle benzénique,

lequel groupe R est substitué en ortho par le groupe R' et porte en plus un autre substituant Y, les deux substituants R' et Y étant fixés sur un atone de carbone du cycle,

R' signifie un groupe carboxy sous forme libre, sous forme d'un sel ou sous forme d'ester ou signifie le groupe CO-$SR^6$ ou CO-$NR^7R^8$ ;

Y signifie H, alkyle en $C_1$-$C_5$, haloalkyle en $C_1$-$C_8$, alcoxy en $C_1$-$C_8$, haloalcoxy en $C_1$-$C_8$, alcényloxy en $C_2$-$C_8$, haloalcényloxy en $C_2$-$C_8$, alcynyloxy en $C_2$-$C_8$, phényle, phénoxy, alkylthio en $C_1$-$C_8$, OH, un halogène, nitro ou cyano ;

$R^2$ signifie le groupe $-C(=NR^{10})-SR^9$ ou $-C(=X)-NHR^{10}$, chaque symbole $R^3$, $R^4$, $R^7$ et $R^8$ signifie indépendamment l'hydrogène ou alkyle en $C_1$-$C_8$ ; chaque symbole $R^6$ et $R^9$ signifie indépendamment alkyle en $C_1$-$C_8$,

$R^{10}$ signifie l'un des groupes

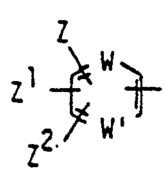

(G-1) ,

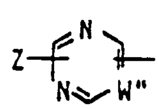

(G-2) ,

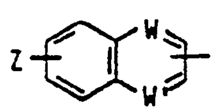

(G-3)

ou

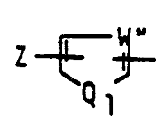

(G-4)

chaque symbole W, W' et W" signifie indépendamment N ou CH,

$Q_1$ signifie l'oxygène, le soufre ou NH,

X signifie l'oxygène ou le soufre,
chaque symbole Z, $Z^1$ et $Z^2$ a indépendamment l'une des significations spécifiées pour Y, mais indépendante de Y,
avec la condition que lorsque R signifie phényle mono-substitué en ortho par le groupe carboxy sous forme libre ou sous forme d'un sel ou par un groupe alcoxycarbonyle en $C_1$-$C_4$, $R^3$ signifie alors alkyle en $C_1$-$C_8$.

2. Un composé de la revendication 1, dans lequel R signifie un groupe 2-COOH-phényle ou 2-$COOR^5$-phényle, lequel groupe phényle est substitué par Y, $R^2$ signifie un groupe –C(=X)-$NHR^{10}$, $R^3$ signifie alkyle en $C_1$-$C_5$, $R^4$ signifie H ou alkyle en $C_1$-$C_5$, $R^5$ signifie alkyle en $C_1$-$C_5$, haloalkyle en $C_1$-$C_5$, alcoxyalkyle en $C_2$-$C_{10}$ ou –CH(-$R^{11}$)-O-C(=O)-alkyle en $C_1$-$C_8$, $R^{10}$ signifie phényle ou pyridyle, lesquels groupes sont substitués par Z et $Z^1$, $R^{11}$ signifie H ou alkyle en $C_1$-$C_5$, X signifie O ou S, chaque symbole Y et Z signifie indépendamment H, un halogène, alkyle en $C_1$-$C_5$, alcoxy en $C_1$-$C_5$, $CF_3$, phényle, phénoxy, nitro, cyano ou hydroxy et $Z^1$ signifie H, un halogène, alkyle en $C_1$-$C_5$ ou alcoxy en $C_1$-$C_5$, tout groupe carboxy dans R étant sous forme libre ou sous forme d'un sel.

3. Un composé de la revendication 1, dans lequel R signifie un cycle hétéroaromatique choisi parmi les cycles

a) pyridyle qui peut être condensé par son côté [b] ou [c] avec un cycle benzénique,
b) 2-pyridyl-N-oxyde ou 2-pyrazinyl-$N^1$-oxyde,
c) pyrimidinyle,
d) pyrazinyle,
e) 3- ou 4-cinnolinyle ou 2-quinoxalinyle et
f) un cycle hétéroaromatique à 5 chaînons fixé par l'un de ses atomes de carbone du cycle au groupe $CR^3$-, choisi parmi les cycles thiényle, furannyle, pyrrolyle, oxazolyle, thiazolyle, isoxazolyle, isothiazolyle, imidazolyle et pyrazolyle ; ledit cycle hétéroaromatique à 5 chaînons pouvant être condensé par son côté [b] avec un cycle benzénique ;

lequel groupe R est substitué en ortho par le groupe R′ et porte en plus un autre substituant Y, les deux substituants R′ et Y étant fixés sur un atome de carbone du cycle ;
R′ signifie un groupe carboxy sous forme libre ou sous forme d'un sel ou signifie le groupe $COOR^5$ ;
$R^3$ signifie H ou alkyle en $C_1$-$C_5$, et $R^2$, $R^4$, $R^5$, $R^{10}$, $R^{11}$, X, Y, Z et $Z^1$ sont tels que spécifiés à la revendication 2.

4. Un composé de la revendication 3, dans lequel le cycle hétéroaromatique R est choisi parmi les cycles pyridyle, quinolyle, 2-pyridyl-N-oxyde, pyrimidinyle, pyrazinyle, thiényle et furyle, substitués par R′ et Y tels que spécifiés à la revendication 3.

5. Un composé de la revendication 4, dans lequel R est choisi parmi pyridyle et thiényle, substitués par R′ et Y tels que spécifiés à la revendication 3 et $R^4$ signifie H.

6. Un composé de la revendication 5, dans lequel $R^3$ signifie alkyle en $C_1$-$C_5$, X signifie l'oxygène, $R^{10}$ signifie phényle substitué par Z, $Z^1$, Z signifie H, un halogène, alkyle en $C_1$-$C_5$, alcoxy en $C_1$-$C_5$ ou $CF_3$ et $Z^1$ signifie H, chloro, fluoro, néthyle ou méthoxy.

7. Un composé de la revendication 6, dans lequel R signifie 2-pyridyle substitué en ortho par R′ tel que spécifié à la revendication 3 et Y signifie H.

8. Un composé de la revendication 6, dans lequel R signifie thiényle substitué en ortho par R′ tel que spécifié à la revendication 3 et Y signifie H.

9. Un composé de la revendication 7, dans lequel $R^{10}$ signifie phényle, 3-chlorophényle ou 3-fluorophényle.

10. Un composé selon l'une quelconque des revendications 1 à 9, dans lequel R′ signifie un groupe carboxy sous forme libre ou sous forme d'un sel ou signifie un groupe COO-CH($R^{11}$)-O-C(=O)-alkyle en $C_1$-$C_5$, où $R^{11}$ est tel que spécifié à la revendication 2.

11. Un composé de la revendication 10 choisi parmi

a) la 4-phénylsemicarbazone de l'acide 2-acétylnicotinique sous forme libre ou sous forme d'un sel,
b) la 4-(3-chlorophényl)semicarbazone de l'acide 2-acétylnicotinique sous forme libre ou sous forme d'un sel,
c) la 4-(3-fluorophényl)-semicarbazone de l'acide 2-acétylnicotinique sous forme libre ou sous forme d'un sel,
d) la 4-(3-fluorophényl)-semicarbazone du 2-acétylnicotinate de butyryloxyméthyle,
e) la 4-(3-chlorophényl)-semicarbazone du 2-acétylnicotinate de butyryloxyméthyle.

12. Un procédé de préparation d'un composé de formule (A) spécifié à la revendication 1, qui comprend

a) la réaction d'un composé de formule I

$$R\text{-}C(=O)\text{-}R^3 \quad I$$

où R et $R^3$ sont tels que définis à la revendication 1, avec un composé de formule II

$$H_2N\text{-}NR^2R^4 \quad II$$

où $R^2$ et $R^4$ sont tels que définis à la revendication 1, suivie, si on le désire, de l'estérification des composés de formule (A) où R' signifie un groupe carboxy en composés (A) où R' signifie un groupe carboxy-ester, ou

b) la S-alkylation d'un composé de formule $A_1$

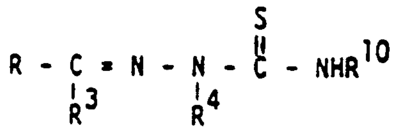

($A_1$)

où R, $R^3$, $R^4$ et $R^{10}$ sont tels que définis à la revendication 1, avec un halogénure d'alkyle de formule III

$$R^9Hal \quad III$$

où $R^9$ est tel que défini à la revendication 1 et Hal signifie un halogène, ou un dérivé fonctionnel réactif dudit composé de formule III, pour obtenir un composé de formule ($A_2$)

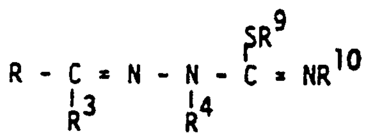

($A_2$)

où R, $R^3$, $R^4$, $R^9$ et $R^{10}$ sont tels que définis à la revendication 1.

13. Une composition herbicide, comprenant un composé de formule (A) selon l'une quelconque des revendications 1 à 11 et un diluant acceptable en agriculture.

14. Un procédé pour combattre les mauvaises herbes, qui comprend l'application sur leur lieu de croissance d'une quantité efficace du point de vue herbicide d'un composé de formule (A) selon l'une quelconque des revendications 1 à 11.

**Revendications pour l'Etat contractant : AT.**

1. Une composition herbicide comprenant un composé de formule (A) :

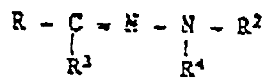

(A)

dans laquelle

R signifie phényle ou naphtyle ou un cycle hétéroaromatique choisi parmi les cycles

a) pyridyle qui peut être condensé par son côté [b] ou [c] avec un cycle benzénique,

b) 2-pyridyl-N-oxyde ou 2-pyrazinyl-$N^1$-oxyde,

c) pyrimidinyle,

d) pyrazinyle,

e) 3- ou 4-cinnolinyle ou 2-quinoxalinyle et

f) un cycle hétéroaromatique à 5 chaînons fixé par l'un de ses atomes de carbone du cycle au groupe $CR^3$, choisi parmi les cycles thiényle, furannyle, pyrrolyle, oxazolyle, thiazolyle, isoxazolyle, isothiazolyle, imidazolyle et pyrazolyle, ledit cycle hétéroaromatique à 5 chaînons pouvant être condensé par son côté [b] avec un cycle benzénique,

lequel groupe R est substitué en ortho par le groupe R' et porte en plus un autre substituant Y, les deux substituant R' et Y étant fixés sur un atome de carbone du cycle,

R' signifie un groupe carboxy sous forme libre, sous forme d'un sel ou sous forme d'ester ou signifie le groupe CO-$SR^6$ ou CO-$NR^7R^8$ ;

Y signifie H, alkyle en $C_1$-$C_5$, haloalkyle en $C_1$-$C_8$, alcoxy en $C_1$-$C_8$, haloalcoxy en $C_1$-$C_8$, alcényloxy en $C_2$-$C_8$, haloalcényloxy en $C_2$-$C_8$, alcynyloxy en $C_2$-$C_8$, phényle, phénoxy, alkylthio en $C_1$-$C_8$, OH, un halogène, nitro ou cyano ;

$R^2$ signifie le groupe —$C(=NR^{10})$-$SR^9$ ou –C(=X)-$NHR^{10}$, chaque symbole $R^3$, $R^4$, $R^7$ et $R^8$ signifie indépendamment l'hydrogène ou alkyle en $C_1$-$C_8$ ; chaque symbole $R^6$ et $R^9$ signifie indépendamment alkyle en $C_1$-$C_8$,

$R^{10}$ signifie l'un des groupes

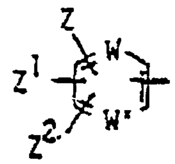 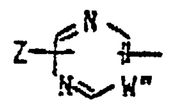 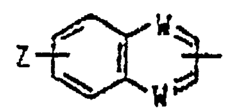 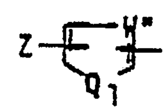

(G-1) , (G-2) , (G-3) (G-4)

chaque symbole W, W' et W" signifie indépendammment N ou CH,

$Q_1$ signifie l'oxygène, le soufre ou NH,

X signifie l'oxygène ou le soufre, chaque symbole Z, $Z^1$ et $Z^2$ a indépendamment l'une des significations spécifiées pour Y, mais indépendante de Y, avec la condition que lorsque R signifie phényle mono-susbtitué en ortho par le groupe carboxy sous forme libre ou sous forme d'un sel ou par un groupe alcoxycarbonyle en $C_1$-$C_4$, $R^3$ signifie alors alkyle en $C_1$-$C_8$.

2. Une composition selon la revendication 1, dans laquelle R signifie un groupe 2-COOH-phényle ou 2-$COOR^5$-phényle, lequel groupe phényle est substitué par Y, $R^2$ signifie un groupe –C(=X)-$NHR^{10}$, $R^3$ signifie alkyle en $C_1$-$C_5$, $R^4$ signifie H ou alkyle en $C_1$-$C_5$, $R^5$ signifie alkyle en $C_1$-$C_5$, haloalkyle en $C_1$-$C_5$, alcoxy-alkyle en $C_2$-$C_{10}$ ou –CH(-$R^{11}$)-O-O(=O)-alkyle en $C_1$-$C_8$, $R^{10}$ signifie phényle ou pyridyle, lesquels groupes sont substitués par Z et $Z^1$, $R^{11}$ signifie H ou alkyle en $C_1$-$C_5$, X signifie O ou S, chaque symbole Y et Z signifie indépendamment H, un halogène, alkyle en $C_1$-$C_5$, alcoxy en $C_1$-$C_5$, $CF_3$, phényle, phénoxy, nitro, cyano ou hydroxy et $Z^1$ signifie H, un halogène, alkyle en $C_1$-$C_5$ ou alcoxy en $C_1$-$C_5$, tout groupe carboxy dans R étant sous forme libre ou sous forme d'un sel.

3. Une composition selon la revendication 1, dans laquelle R signifie un cycle hétéroaromatique choisi parmi les cycles

a) pyridyle qui peut être condensé par son côté [b] ou [c] avec un cycle benzénique,
b) 2-pyridyl-N-oxyde ou 2-pyrazinyl-$N^1$-oxyde,
c) pyrimidinyle,
d) pyrazinyle,
e) 3- ou 4-cinnolinyle ou 2-quinoxalinyle et
f) un cycle hétéroaromatique à 5 chaînons fixé par l'un de ses atomes de carbone du cycle au groupe $CR^3$, choisi parmi les cycles thiényle, furannyle, pyrrolyle, oxazolyle, thiazolyle, isoxazolyle, isothiazolyle, imidazolyle et pyrazolyle ; ledit cycle hétéroaromatique à 5 chaînons pouvant être condensé par son côté [b] avec un cycle benzénique ;

lequel groupe R est substitué en ortho par le groupe R' et porte en plus un autre substituant Y, les deux substituants R' et Y étant fixés sur un atome de carbone du cycle ;

R' signifie un groupe carboxy sous forme libre ou sous forme d'un sel ou signifie le groupe $COOR^5$ ;

$R^3$ signifie H ou alkyle en $C_1$-$C_5$, et

$R^2$, $R^4$, $R^5$, $R^{10}$, $R^{11}$, X, Y, Z et $Z^1$ sont tels que spécifiés à la revendication 2.

4. Une composition selon la revendication 3, dans laqelle le cycle hétéroaromatique R est choisi parmi les cycles pyridyle, quinolyle, 2-pyridyl-N-oxyde, pyrimidinyle, pyrazinyle, thiényle et furyle, substitués par R' et Y tels que spécifiés à la revendication 3.

5. Une composition selon la revendication 4, dans laquelle R est choisi parmi pyridyle et thiényle, substitués par R' et Y tels que spécifiés à la revendication 3 et $R^4$ signifie H.

6. Une composition selon la revendication 5, dans laquelle $R^3$ signifie alkyle en $C_1$-$C_5$, X signifie l'oxygène, $R^{10}$ signifie phényle substitué par Z, $Z^1$, Z signifie H, un halogène, allyle en $C_1$-$C_5$, alcoxy en $C_1$-$C_5$ ou $CF_3$ et $Z^1$ signifie H, chloro, fluoro, méthyle ou méthoxy.

7. Une composition selon la revendication 6, dans laquelle R signifie 2-pyridyle substitué en ortho par R' tel que spécifié à la revendication 3 et Y signifie H.

8. Une composition selon la revendication 6, dans laquelle R signifie thiényle substitué en ortho par R' tel que spécifié à la revendication 3 et Y signifie H.

9. Une composition selon la revendication 7, dans laquelle $R^{10}$ signifie phényle, 3-chlorophényle ou 3-fluorophényle.

10. Une composition selon l'une quelconque des revendications 1 à 9, dans laquelle R' signifie un groupe carboxy sous forme libre ou sous forme d'un sel au signifie un groupe COO-CH($R^{11}$)-O-C(=O)-alkyle en $C_1$-$C_5$, où $R^{11}$ est tel que spécifié à la revendication 2.

11. Une composition selon la revendication 10 comprenant un composé choisi parmi

a) la 4-phénylsemicarbazone de l'acide 2-acétylnicotinique sous forme libre ou sous forme d'un sel,

b) la 4-(3-chlorophényl)semicarbazone de l'acide 2-acétylnicotinique sous forme libre ou sous forme d'un sel,

c) la 4-(3-fluorophényl)-semicarbazone de l'acide 2-acétylnicotinique sous forme libre ou sous forme d'un sel,

d) la 4-(3-fluorophényl)-semicarbazone du 2-acétylnicotinate de butyryloxyméthyle,

e) la 4-(3-chlorophényl)-semicarbazone du 2-acétylnicotinate de butyryloxyméthyle.

12. Un procédé de préparation d'un composé de formule (A) spécifié à la revendication 1, qui comprend

a) la réaction à un composé de formule I

$$R\text{-}C(=O)\text{-}R^3 \quad I$$

où R et $R^3$ sont tels que définis à la revendication 1, avec un composé de formule II

$$H_2N\text{-}NR^2R^4 \quad II$$

où $R^2$ et $R^4$ sont tels que définis à la revendication 1, suivie, si on le désire, de l'estérification des composés de formule (A) où R' signifie un groupe carboxy en composés (A) où R' signifie un groupe carboxy-ester, ou

b) la S-alkylation d'un composé de formule $A_1$

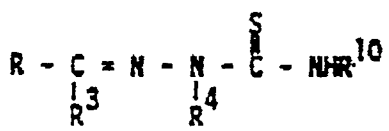

($A_1$)

où R, $R^3$, $R^4$ et $R^{10}$ sont tels que définis à la revendication 1, avec un halogénure d'alkyle de formule III

$$R^9Hal \quad III$$

où $R^9$ est tel que défini a la revendication 1 et Hal signifie un halogène, ou un dérivé fonctionnel réactif dudit composé de formule III, pour obtenir un composé de formule ($A_2$)

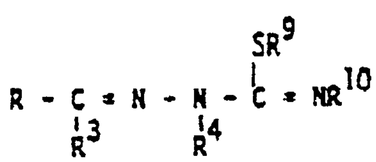

($A_2$)

où R, $R^3$, $R^4$, $R^9$ et $R^{10}$ sont tels que définis à la revendication 1.